(19) Europäisches Patentamt
European Patent Office
Office européen des brevets

(11) Publication number: **0 010 396**
**A1**

(12)
# EUROPEAN PATENT APPLICATION

(21) Application number: 79302156.9

(22) Date of filing: 09.10.79

(51) Int. Cl.³: **A 01 N 37/34**
//C07C121/28, C07C121/407,
C07C121/417, C07C121/45

(30) Priority: 24.10.78 GB 4168978
09.02.79 GB 7904657
17.08.79 GB 7928705

(43) Date of publication of application:
30.04.80 Bulletin 80/9

(84) Designated Contracting States:
AT BE CH DE FR GB IT LU NL SE

(71) Applicant: **FISONS LIMITED**
**Fison House 9 Grosvenor Street**
**London(GB)**

(72) Inventor: **Judson, Philip Neville**
**27 Finchams Close**
**Linton, Cambridge(GB)**

(72) Inventor: **White, Charles Richard Hart**
**110 High Street**
**Harston, Cambridge(GB)**

(74) Representative: **Craig, Christopher Bradberry et al,**
**Fisons Limited Fison House Princes Street**
**Ipswich 1P1 1QH(GB)**

(54) **Fungicidal and herbicidal compositions, certain cyanomethane and cyanoethene derivatives being active agents thereof, the preparation of these derivatives and methods for combating fungi and weeds.**

(57) There are described pesticidal compositions comprising a compound of formula I,

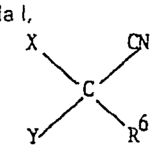

in which X represents a group -CN and Y a cation, or X and Y together form a group of formula II,

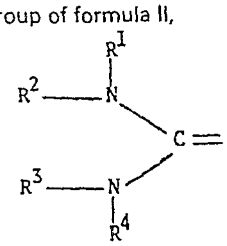

in which $R^1$ and $R^2$, which may be the same or different, each represents hydrogen, alkyl, cycloalkyl or benzyl,

$R^3$ and $R^4$, which may be the same or different, each represent hydrogen, alkyl, cycloalkyl, phenyl or phenyl substituted by one or more of halogen, alkyl, alkoxy, phenoxy, nitro, amino, -COOR⁷, -SO₂R⁷ or -CF₃, or $R^3$ and $R^4$ together form a 4, 5 or 6 membered methylene chain which may be fused to a benzene ring, or which may be interrupted by an oxygen atom or by a group -NH-,

$R^6$ is -CN, -C(=NH)-NHR$^8$, -CONR$^9$R$^{10}$, or -CQZR$^9$,

$R^8$ represents alkyl or cycloalkyl,

$R^9$ and $R^{10}$, which may be the same or different, each represent hydrogen, alkyl, phenyl or cycloalkyl, and

Q and Z, which may be the same or different, each represent oxygen or sulphur,

or a suitable derivative thereof,

provided that when $R^6$ is $-COOC_2H_5$ and $R^1$, $R^2$ and $R^3$ are all hydrogen, then $R^4$ is other than alkyl containing 3, 4 or 5 carbon atoms,

together with an agriculturally acceptable adjuvant, diluent or carrier.

There are also described methods for making and using the compounds. Certain of the compounds are new.

EP 0 010 396 A1

BAD ORIGINAL

TITLE MODIFIED
see front page

0010396

[illegible] METHANES, METHODS FOR THEIR PRODUCTION AND [illegible] AND PESTICIDAL COMPOSITIONS CONTAINING THEM

The present invention relates to new fungicidal and/or herbicidal compounds, fungicidal and/or herbicidal compositions and methods of combating fungus or weeds.

Accordingly the present invention provides a fungicidal or herbicidal composition comprising a compound of formula I,

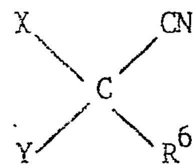

I

in which X represents a group -CN and Y a cation, or X and Y together form a group of formula II,

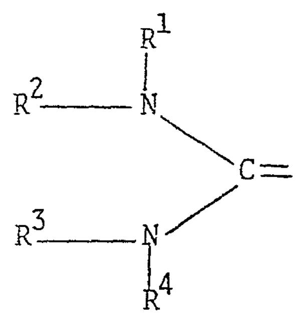

II

in which $R^1$ and $R^2$, which may be the same or different, each represents hydrogen, alkyl, cycloalkyl or benzyl,

$R^3$ and $R^4$, which may be the same or different, each represent hydrogen, alkyl, cycloalkyl, phenyl or phenyl substituted by one or more of halogen, alkyl, alkoxy, phenoxy, nitro, amino, $-COOR^9$, $-SO_3R^9$ or $-CF_3$, or $R^3$ and $R^4$ together form a 4, 5 or 6 membered methylene chain which may be fused to a benzene ring, or which may be interrupted



BAD ORIGINAL

by an oxygen atom or by a group -NH-,

$R^6$ is -CN, $-C(=NH)-NHR^8$, $-CONR^9R^{10}$, or $-CQZR^9$,

$R^8$ represents alkyl or cycloalkyl,

$R^9$ and $R^{10}$, which may be the same or different, each represent hydrogen, alkyl, phenyl or cycloalkyl, and

Q and Z, which may be the same or different, each represent oxygen or sulphur,

or a suitable derivatives thereof,

provided that (a) when $R^6$ is $-COOC_2H_5$ and $R^1$, $R^2$ and $R^3$ are all hydrogen, then $R^4$ is other than alkyl containing 3, 4 or 5 carbon atoms,

together with an agriculturally acceptable adjuvant, diluent or carrier, e.g. a surface active agent, a pesticide (for example a herbicide, insecticide, fungicide, acaricide or the like), a solid carrier, or a liquid carrier, e.g. a hydrocarbon having a boiling point in the range 130-270°C.

In a further embodiment, the invention provides a method for combating fungus or weeds which comprises applying a suitable compound of formula I, or a suitable derivative thereof, to a locus infested, or liable to be infested, by fungal attack or by weeds.

According to the invention we also provide, as new compounds, the compounds of formula I, and suitable derivatives thereof, with the provisos that

A. when X and Y together form a group of formula II,

and (b) when $R^6$ is -CN

(i) then not all of $R^1$, $R^2$, $R^3$ and $R^4$ are hydrogen,

(ii) then not all of $R^1$, $R^2$, $R^3$ and $R^4$ are methyl,

(iii) and when $R^1$ and $R^3$ are both hydrogen, then both of $R^2$ and $R^4$ are not octadecyl, or both of $R^2$ and $R^4$ are not p-nitrophenyl,

(iv) and when $R^1$, $R^2$ and $R^3$ are all hydrogen, then $R^4$ is not methyl or cyclohexyl,

(v) and when $R^1$ and $R^2$ are both methyl, then $R^3$ and $R^4$ are not both ethyl or propyl or do not together form a $-(CH_2)_2-O-(CH_2)_2-$ chain, and, when $R^3$ is hydrogen, $R^4$ is not butyl, phenyl or p-chlorophenyl,

(vi) and when $R^1$ and $R^2$ are both hydrogen, then $R^3$ and $R^4$ are not both ethyl or propyl or do not together form a $-(CH_2)_4-$, or a $-(CH_2)_5-$ chain, and, when $R^3$ is hydrogen or methyl, then $R^4$ is not hydrogen, ethyl, butyl, phenyl, o-methylphenyl or m-nitrophenyl,

(vii) and when $R^3$ and $R^4$ are both hydrogen and $R^1$ is hydrogen or ethyl, then $R^2$ is not benzyl, and

(c) when $R^6$ is $-COOCH_3$

(i) not all of $R^1$, $R^2$, $R^3$ and $R^4$ are methyl,

(ii) and when $R^1$, $R^2$ and $R^3$ are all hydrogen, then $R^4$ is not alkyl C 1 to 6, cyclohexyl or phenyl,

(iii) and when $R^1$, $R^3$, and $R^4$ are all hydrogen, then $R^2$ is not benzyl, and

(d) when $R^6$ is $-COOC_2H_5$

(i) and when $R^1$, $R^2$ and $R^3$ are all hydrogen, then $R^4$ is not methyl



BAD ORIGINAL

or alkyl C 3 to 6 inclusive,

(ii) and when $R^1$ and $R^2$ are both hydrogen, then $R^3$ and $R^4$ do not together form a $-(CH_2)_4-$ chain, and

(e) when $R^6$ is $-CONH_2$, then not all of $R^1$, $R^2$, $R^3$ and $R^4$ are hydrogen, and

(f) when $R^6$ is $-COOCH(CH_3)_2$ or $-COOC(CH_3)_3$ and $R^1$, $R^2$ and $R^3$ are all hydrogen, then $R^4$ is not propyl, butyl or hexyl, and

(g) when $R^6$ is -CONHphenyl not all of $R^1$, $R^2$, $R^3$ and $R^4$ are ethyl,

or

B. when X and $R^6$ are -CN then Y is an organic quaternary ammonium cation other than tetramethylammonium, or is a cation comprising one or more hydrogen atoms co-ordinated to an organic base other than isopropylamine, t-butylamine, t-octylamine, diethylamine, triethylamine, pyridine, piperidine, pyrrolidine, benzamidine, aniline, N-methylaniline, o-toluidine or m-nitroaniline.

According to the invention we also provide a process for the production of the new compounds of formula I, and suitable derivatives thereof, which comprises

(a) production of a compound of formula I in which X and Y together form a group of formula II by reacting a compound of formula III,

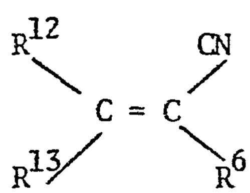

III

in which at least one of $R^{12}$ and $R^{13}$ is a leaving group, and the other of $R^{12}$ and $R^{13}$ may be $-NR^3R^4$ or $-NR^1R^2$ respectively,

with an amine of formula IV or V,

$R^3R^4NH$ IV

$R^1R^2NH$ V

in which $R^1$, $R^2$, $R^3$, $R^4$, $R^6$ and the provisos are as defined above, or

(b) production of a compound of formula I in which at least one of $R^1$, $R^2$, $R^3$ and $R^4$ represents alkyl, by alkylation of a corresponding compound of formula I in which at least one of $R^1$, $R^2$, $R^3$ and $R^4$ represents hydrogen, or

(c) production of a compound of formula I in which $R^3$ and $R^4$ or $R^1$ and $R^2$ are both hydrogen by subjecting to an elevated temperature a compound of formula VI or VII,

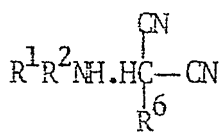 VI

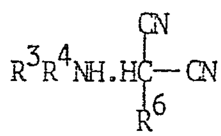 VII

in which $R^1$, $R^2$, $R^3$, $R^4$, $R^6$ and the provisos are as defined above,

(d) production of a compound of formula I in which X and Y together form a group of formula II and $R^6$ is $-CONH_2$, by selective hydrolysis of the corresponding compound of formula I in which $R^6$ is -CN,

(e) production of a compound of formula I in which X and Y together form a group of formula II and $R^6$ is $-C(=NH)-NHR^8$, by reacting a



BAD ORIGINAL

corresponding compound of formula I or III in which $R^6$ is -CN, with an amine of formula $R^8NH_2$ in which $R^8$ is as defined above, or

(f) production of a compound of formula I in which X is -CN and Y is a cation, by a metathetical process,

and where desired or necessary converting the resulting compound of formula I to a suitable derivative thereof or *vice versa*.

Process (a) may be carried out in a solvent which is inert under the reaction conditions, e.g. water, 2-methoxyethanol or a lower alkanol such as ethanol, or a mixture thereof. The reaction preferably takes place at an elevated temperature, e.g. of from about 50° to 130°C. We prefer $R^{12}$ or $R^{13}$, when they are a leaving group to be $-CCl_3$, alkoxy, halogen, e.g. chlorine or bromine, or more preferably -S alkyl, e.g. -S alkyl C 1 to 6.

The alkylation of process (b) may be effected by using an alkyl halide, e.g. an alkyl iodide or bromide, or an alkyl sulphate. The reaction is preferably carried out in the presence of an acid acceptor, e.g. potassium carbonate, and if desired a catalytic amount of potassium iodide may be present. The reaction may be carried out in a solvent which is inert under the reaction conditions, e.g. acetone. The reaction is preferably carried out at temperature of from 20 to 100°C.

Process (c) may be carried out by heating the compound of formula VI or VII in a reaction medium which is inert under the reaction conditions, e.g. dilute hydrochloric acid, water or xylene.



BAD ORIGINAL

or in an amine $R^1R^2NH$ or $R^3R^4NH$. The reaction is preferably carried out at a temperature of from 60 to 160°C.

Process (d) may be carried out under neutral, acidic (e.g. 80% $H_2SO_4$) or basic conditions, e.g. in the presence of sodium hydroxide. The reaction may be carried out at from about 20° to 100°C.

Process (e) may be carried out at an elevated temperature, e.g. of from 60° to 160°C. The reaction is preferably carried out in a solvent which is inert under the reaction conditions, e.g. an alkanol such as ethanol.

Process (f) may be carried out by the reaction of a solution of a metal salt of the cyanomethane (for example potassium tricyanomethanide) with a salt of the quaternary ammonium cation or the organic base. Preferred quaternary ammonium salts are the halides, particularly the bromides and chlorides.

Compounds of formula III are either known or can be made from known compounds using techniques known <u>per se</u>, e.g. using the following reaction scheme:-

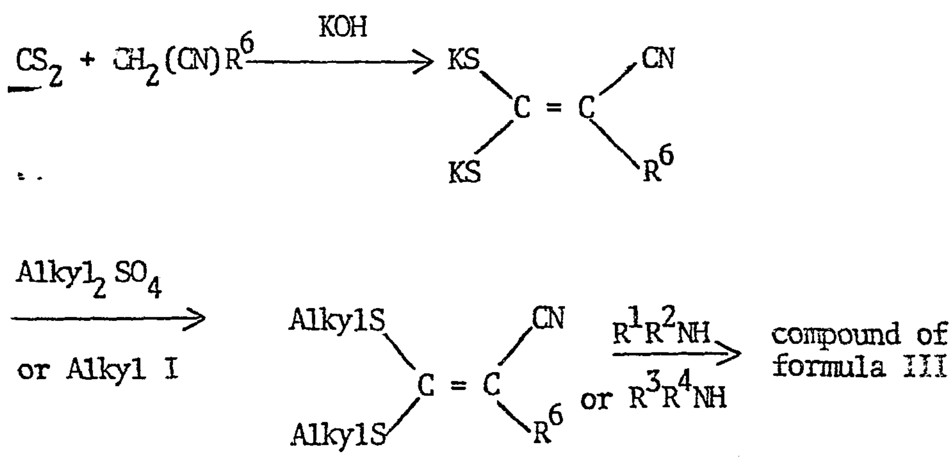


BAD ORIGINAL

In the above reaction scheme $R^1$, $R^2$, $R^3$, $R^4$ and $R^6$ are as defined above.

Compounds of formula VI and VII may be made by reacting a compound of formula IV or V, or an acid addition salt thereof, with a compound of formula VIII,

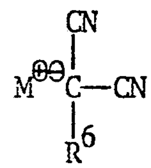

VIII

in which $R^6$ is as defined above, and

$M^{\oplus}$ is a metallic ion, e.g. an alkali metal ion.

Compounds of formulae IV, V and VIII are either known or may be made from known compounds using techniques which are known per se.

Preferred salts of formula I are of formula Ia,

$$M\left[\ C(CN)_3\ \right]_n \qquad Ia$$

wherein M is a metal, for example an alkali metal such as sodium or potassium, an alkaline earth metal such as magnesium or calcium, or a heavy metal such as zinc, copper or iron, ammonium, organic quaternary ammonium, or one or more hydrogen atoms co-ordinated to an organic base, for example a primary, secondary or tertiary amine, guanidine or substituted guanidine, or a heterocyclic compound having a nitrogen-containing ring such as a substituted or unsubstituted pyridine, piperidine, pyrrolidine, imidazole, morpholine,



BAD ORIGINAL

triazole, or quinoxaline group, and n is the valency of the group M. The substituted guanidine may for instance be guanidine substituted, preferably monosubstituted, by alkyl, e.g. of 1 to 16, preferably 8-16, carbon atoms.

A preferred group of new salts are those of formula Ib,

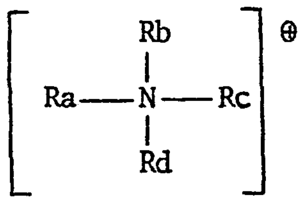

$C(CN)_3^{\ominus}$ Ib

wherein Ra is alkyl of 2 to 20, preferably 12 to 17, carbon atoms, benzyl or substituted benzyl, and Rb, Rc and Rd, which may be same or different, are alkyl of 1 to 4 carbon atoms, e.g. methyl. The benzyl group is suitably substituted by one or more alkyl groups of 1 to 4 carbon atoms, which alkyl groups may be substituted, for example by halogen e.g. chlorine or bromine, thiocyanate or trialkylammonium.

Preferred salts are those of formula Ib (e.g. the hexadecyltrimethyl ammonium salt of tricyanomethane), and the piperidine, morpholine, and especially the dodecylguanidine salt of tricyanomethane.

We prefer compounds of formula I in which X and Y together form a group of formula II.

In the compounds of formula I we prefer each of $R^1$, $R^2$, $R^3$, $R^4$ and $R^6$, when they contain carbon, to contain less than 25, preferably less than 15, more preferably less than 10, and most preferably less than 8, carbon



BAD ORIGINAL

atoms. We particularly prefer $R^4$ to be branched alkyl C 3 to 6, phenyl or phenyl substituted by one or more or C 1 to 6 alkyl groups, $-NH_2$ or $-CF_3$ groups, or halogen, e.g. bromine, chlorine or iodine, atoms. $R^3$ is preferably hydrogen or alkyl C 1 to 6, e.g. methyl. $R^1$ and $R^2$ are preferably chosen from hydrogen or alkyl C 1 to 6. When one of $R^1$, $R^2$, $R^3$, $R^4$ or $R^6$ contains a cycloalkyl group we prefer the cycloalkyl group to be cyclopentyl or cyclohexyl. $R^6$ is preferably -CN or $-C(=NH)-NHCH_3$, or more preferably a carboxylic acid group, or a C 1 to 20, e.g. a C 1 to 6, ester or C 1 to 6 alkyl substituted, or an unsubstituted amide thereof.

As a specific group we provide compounds of formula I in which $R^4$ is phenyl optionally substituted by chlorine, bromine, iodine, methyl, $-CF_3$ or $-NH_2$, $R^3$ is hydrogen or methyl, $R^1$ and $R^2$ are chosen from hydrogen or methyl, and $R^6$ is -CN, $-CONH_2$, $-COOC_2H_5$, -COO*i*-propyl, -COO*t*-butyl or $-COOCH(CH_3)CH_2CH_3$. We also provide the same group, but in which $R^6$ is not -CN.

For enhanced herbicidal activity we prefer those compounds of formula I in which $R^4$ is as defined immediately above, $R^3$ is an alkyl group, e.g. a methyl group, and $R^6$ is an ester, e.g. an ethyl, propyl or butyl ester, of a carboxylic acid group. For enhanced fungicidal activity we prefer compounds of formula I in which $R^1$ and $R^2$ are hydrogen, $R^3$ is hydrogen or alkyl, $R^4$ is phenyl substituted by chlorine, bromine and/or methyl, and $R^6$ is -CN or preferably $-CONH_2$.

Thus preferred compounds of formula I are of formula Ia,



BAD ORIGINAL

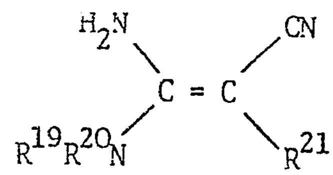

Ic

in which $R^{19}$ is hydrogen or methyl,

$R^{20}$ is a group $R^{16}$ or a group

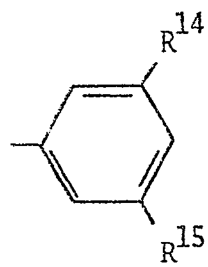

or

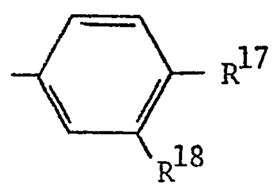

$R_{21}$ is $-CONH_2$ or $-COOR$,

R is straight or branched alkyl C 2 to 12,

$R^{14}$ and $R^{15}$, which may be the same or different, are each hydrogen, methyl, $-CF_3$ or nitro,

$R^{16}$ is branched chain alkyl C 3 to 6,

$R^{17}$ is chlorine or bromine, and

$R^{18}$ is hydrogen or methyl.

Particularly preferred compounds of formula Ic are of formula Id, Ie and If,

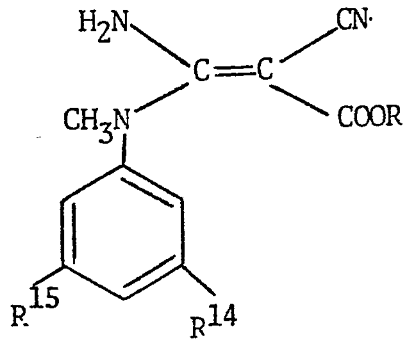

Id

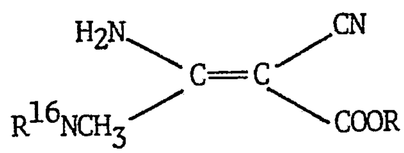

Ie

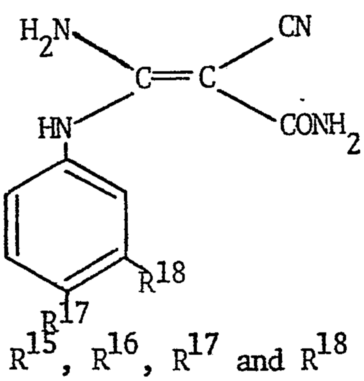

If

in which R, $R^{14}$, $R^{15}$, $R^{16}$, $R^{17}$ and $R^{18}$ are as defined above.

As a further specific group we provide those compounds of formula I in which $R^1$ and $R^2$ are both hydrogen, $R^3$ is methyl, $R^4$ is phenyl and $R^6$ is -COOH or -COSH or a branched C 2 to 7 alkyl ester thereof.

The compounds of Examples 11(b), 11(c), 11(d), 18(b), 18(c) and 43 are particularly preferred.

Suitable derivatives of the compounds of formula I in which X and Y together form a group of formula II include salts, e.g. in certain cases salts with alkali metal or alkaline earth metals or with organic bases, and in certain cases with organic or inorganic acids. The derivatives may be made from the compounds of formula I in ways which are known per se.

Certain of the compounds of formula I may exist in stereoisomeric form and both isomeric forms of such compounds are included within the scope of this invention, i.e. where X and Y together form a group of formula II and the groups $R^6$ and -CN are interchanged. In general the isomeric forms interchange readily and we prefer that isomeric form which is most stable at room temperature.

The present compounds are normally employed in the form of compositions, which can be prepared by admixing the ingredients. Usually the compositions are initially produced in the form of concentrates, e.g. containing 0.5-85% of the compound of formula I, and these are diluted with water or hydrocarbon, usually water, for application, generally such that the concentration of the compound is 0.05-5%, though in ultra low volume application the concentration may be higher, e.g. up to 20%. Percentages and parts in this specification are by weight unless otherwise indicated.

The compositions normally contain a surface active agent and/or a carrier.

The carrier may be a liquid, e.g. water (e.g. water used to dilute a concentrate for application). If water is employed as carrier in a concentrate, an organic solvent may also be present as carrier, though this is not usually employed. A surface active agent may advantageously be present.

Those compounds soluble in water may be used as aqueous solutions with or without a surface active agent.

The carrier may be a liquid other than water, for example an organic solvent, such as a water immiscible solvent, e.g. a hydrocarbon which boils within the range 130-270°C, in which the compound is dissolved or suspended. A concentrate containing a water immiscible solvent suitably also contains a surface active agent so that the concentrate acts as a self-emulsifiable oil on admixture with water. The liquid may be a water-miscible solvent e.g. 2-methoxy ethanol, methanol, propylene glycol, diethylene glycol, diethylene glycol monoethyl ether, formamide or methylformamide.

The carrier may be a solid, which may be finely divided. Examples ~~of~~ suitable solids are limestone, clays, sand, mica, chalk, attapulgite, diatomite, perlite, sepiolite, silicas, silicates, lignosulphonates, peat and solid fertilizers. The carrier can be of natural or synthetic origin or can be a modified natural material.

Wettable powders soluble or dispersable in water may be formed by admixing the compound in particulate form with a



BAD ORIGINAL

particulate carrier or spraying molten compound on to the particulate carrier, admixing a wetting agent and a dispersing agent and finely grinding the whole powder mixture.

An aerosol composition may be formed by admixing the compound with a propellant, e.g. a polyhalogenated alkane such as dichlorodifluoromethane, and suitably also with a solvent.

A flowable suspension concentrate may be formed if the compound has a low water solubility by grinding the compound with water, a wetting agent and a suspending agent.

A flowable suspension concentrate wherein the carrier is a hydrocarbon which boils within the range 130-270$^{o}$C rather than water may be formed.

Thus the present composition can for example be solid (e.g. dust or granules) and contain a solid carrier or liquid (e.g. an emulsifiable concentrate) and contain a liquid carrier which is a hydrocarbon which boils within the range 130-270$^{o}$C.

The term 'surface active agent' is used in the broad sense to include materials variously called emulsifying agents, dispersing agents and wetting agents. Such agents are well known in the art.

The surface active agents used may comprise anionic surface active agents, for example soaps, mono- or di-esters of phosphoric acid with fatty alcohol ethoxylates or salts of such esters, fatty alcohol sulphates such as sodium dodecyl sulphate, sodium octadecyl sulphate or sodium cetyl sulphate, ethoxylated fatty

alcohol sulphates, ethoxylated alkylphenol sulphates, lignin sulphonates, petroleum sulphonates, alkyl-aryl sulphonates such as alkyl-benzene sulphonates or lower alkylnaphthalene sulphonates e.g. butyl-naphthalene sulphonate, salts of sulphonated naphthaleneformaldehyde condensates, salts of sulphonated phenol-formaldehyde condensates, or more complex sulphonates such as the amide sulphonates e.g. the sulphonated condensation product of oleic acid and N-methyl taurine or the dialkyl sulphosuccinates e.g. the sodium sulphonate of dioctyl succinate. Ionic surface active agents may tend to result in precipitation if employed in some formulations with certain of the compounds of formula I. Any surface active agent should be so chosen of course as to avoid this for any particular formulation envisaged.

The surface active agents may also comprise non-ionic agents, for example condensation products of fatty acid esters, fatty alcohols, fatty acid amides or fatty-, alkyl- or alkenyl-substituted phenols with ethylene oxide, fatty esters of polyhydric alcohol ethers e.g. sorbitan fatty acid esters, condensation products of such esters with ethylene oxide e.g. polyoxyethylene sorbitan fatty acid esters, block copolymers of ethylene oxide and propylene oxide, acetylenic glycols such as 2,4,7,9-tetramethyl-5-decyn-4,7-diol, or ethoxylated acetylenic glycols.

The surface active agents may also comprise cationic agents, for example alkyl- and/or aryl-substituted quaternary ammonium compounds such as cetyl trimethylammonium bromide, ethoxylated

tertiary fatty amines or a compound containing a cation as shown in formula Ib above. A surface active agent containing a cation as in formula Ib may advantageously be used with a salt of formula Ib containing the same cation.

Preferred surface active agents include ethoxylated fatty alcohol sulphates, lignin sulphonates, alkyl-aryl sulphonates, salts of sulphonated naphthalene-formaldehyde condensates, salts of sulphonated phenol-formaldehyde condensates, dialkyl sulphosuccinates, alkyl phenol ethoxylates, and fatty alkyl ethoxylates.

Non-ionic surface active agents are preferred.

Higher quantities of surface active agent, e.g. 5-50% of concentrate, than is normally present in commercial pesticidal or plant growth regulant compositions have been found to increase considerably the activity of the present compounds, even in some cases to several times the original activity.

The surface active agent employed to produce this potentiating effect may be selected from those described above. It is preferably a non-ionic surface active agent, especially an alkylsubstituted phenol condensed with ethylene oxide, e.g. tributyl-phenol condensed with 11 moles of ethylene oxide (available under the trade mark Sapogenat T110). The potentiating surface active agent may be admixed with the present compound for instance at the point of use, e.g. in a spray tank, or before, e.g. in a concentrate. Preferably the amount of potentiating surface active



BAD ORIGINAL

agent applied in a spray of the present compound is 0.1-5%, especially 1%.

The present active compound may be admixed with one or more other pesticides, e.g. herbicides, insecticides or fungicides, or with a plant growth regulant. The invention provides a one pack presentation, in which the present compound is already mixed with another pesticide or plant growth regulant, and also a single package designed to hold the present compound and other pesticide or the plant growth regulant in separate containers, for mixing, e.g. in a spray tank, for application. Particular advantages are obtained with mixtures with another pesticide. The present compound may be used sequentially with one or more other pesticides or plant growth regulants particularly with another fungicide or herbicide.

The herbicide may be for example one or more of a phenoxyaliphatic acid, substituted urea, triazine, phenol, nitrile, bipyridylium compound, substituted benzoic acid, halogenated aliphatic acid, carbamate, thiocarbamate, chloroacetamide, diazine, benzofuran or arsenic herbicide. In respect of selective herbicidal compositions for post-emergence use, the present compound may be used in admixture with for example a substituted phenoxyaliphatic acid; in respect of selective herbicidal compositions for pre-emergence use, the present compound may be used in admixture with for example a substituted urea, triazine, S-2,3-dichloroallyl di-isopropylthiocarbamate or S-2,3,3-trichloroallyl di-isopropyl-

BAD ORIGINAL

thiocarbamate.

The phenoxyaliphatic acid generally comprises alkyl and/or halogen substituted phenoxyaliphatic acids, and their salts, for example alkali metal, amine and alkanolamine salts, and functional derivatives, for example esters and amides. These compounds may be of activity such that they are recognised as commercial herbicides, or may be of only slight herbicidal activity. Examples of the substituted phenoxyaliphatic acids which may be mentioned include 2,4-dichlorophenoxyacetic acid, 2-(2,4-dichlorophenoxy)propionic acid, 2-methyl-4-chlorophenoxyacetic acid, 2,4,5-trichlorophenoxyacetic acid, gamma-2,4-dichlorophenoxybutyric acid, gamma-2-methyl-4-chloro-phenoxybutyric acid, alpha-2-methyl-4-chlorophenoxypropionic acid, 2-(4-[2,4-dichlorophenoxy] phenoxy)propionic acid and 2-(4-[4-chlorophenoxy]phenoxy) propionic acid.

The substituted urea generally comprises a tri- or tetra-substituted urea such as N'-(3-chloro-4-methoxyphenyl)-N,N-dimethylurea, N'-(3-chloro-4-methylphenyl)-N,N-dimethylurea, N'-parachlorophenyl-N,N-dimethylurea, N-butyl-N'-(3,4-dichlorophenyl)-N-methylurea, N'-parachlorophenyl-O,N,N-trimethylisourea, N'-p-chlorophenyl-N-methoxy-N-methylurea, N,N-dimethyl-N'-phenylurea, 3-(4-bromophenyl)-1-methoxy-1-methylurea, 1-(2-benzothiazolyl)-3-methylurea, N,N-dimethyl-N'-(4-[1-methylethyl]phenyl) urea, N'-(3,4-dichlorophenyl)-N-methoxy-N-methylurea, N,N-dimethyl-N'-[3-(trifluoromethyl)phenyl]urea, N'-(3,4-dichlorophenyl)-N,N-dimethylurea or N'-(3-(1,1,2,2-tetrafluoroethoxy)phenyl)-N,N-dimethylurea.

The triazine herbicide generally comprises 2-chloro-4-(1-cyano-1-methylamino)-6-ethylamino-1,3,5-triazine or 2-isopropylamino-4-(3-methoxypropylamino)-6-methylthio-1,3,5-triazine or a compound of the formula:-

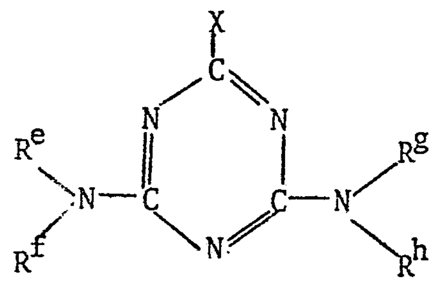

where X is a halogen atom, OY group or SY group, where Y is an alkyl group, $R^e$ and $R^g$ are the same or different and are hydrogen or alkyl and $R^f$ and $R^h$ are the same or different alkyl groups, such as 2-chloro-4,6-bisethylamino-1,3,5-triazine, 2-chloro-4-ethylamino-6-diethylamino-1,3,5-triazine, 2-chloro-6-ethylamino-4-isopropylamino-1,3,5-triazine or 2,4-bis(isopropylamino)-6-methylthio-1,3,5-triazine.

The phenol herbicide generally comprises 4,6-dinitro-o-cresol, 4,6-dinitro-2-sec-butylphenol or pentachlorophenol. The nitrile herbicide generally comprises 3,5-diiodo-4-hydroxy-benzonitrile, 3,5-dibromo-4-hydroxybenzonitrile or 2,6-dichlorobenzonitrile. The bipyridylium herbicide generally comprises 1,1'-dimethyl-4,4'-bipyridylium dichloride or 1,1'-ethylene-2,2'-bipyridylium dibromide. The substituted benzoic acid herbicide generally



BAD ORIGINAL

comprises 2,3,6-trichlorobenzoic acid, 2-methoxy-3,6-dichlorobenzoic acid or N-(1,1-dimethylpropynyl)-3,5-dichlorobenzamide. The halogenated aliphatic acid herbicide generally comprises trichloroacetic acid or 2,2-dichloropropionic acid. The carbamate herbicide generally comprises isopropyl N-(3-chlorophenyl) carbamate, 4-chloro-2-butynyl N-(3-chlorophenyl)carbamate, methyl 3-(m-tolylcarbamoyloxy)phenylcarbamate, isopropyl N-(3-(N-ethyl-N-phenylcarbamoyloxy)phenyl)carbamate, or D-N-ethyl-2-(phenylcarbamoyloxy)propionamide. The thiocarbamate herbicide generally comprises S-ethyl N,N-dipropylthiocarbamate, S-ethyl N,N-diisobutylthiocarbamate, S-(2,3-dichloroallyl) N,N-diisopropylthiocarbamate, S-ethyl N-ethyl-N-cyclohexylthiocarbamate, S-propyl butylethylthiocarbamate or S-(2,3,3-trichloroallyl) N,N-diisopropylthiocarbamate. The chloroacetamide herbicide generally comprises N,N-diallyl-2-chloroacetamide, N-isopropyl-2-chloroacetanilide, N-chloroacetyl-N-(2,6-diethylphenyl)glycine ethyl ester, N-(2,6-diethylphenyl)-N-(methoxymethyl)-2-chloroacetamide, N-(2-ethyl-6-methylphenyl)-N-(2-methoxy-1-methylethyl)-2-chloroacetamide, or N-chloroacetyl-N-(2-methyl-6-ethylphenyl)glycine isopropyl ester. The diazine herbicide generally comprises 5-bromo-6-methyl-3-sec-butyluracil, 3-cyclohexyl-5,6-trimethyleneuracil, 5-amino-4-chloro-2-phenyl-3-pyridazinone or 1,2-dihydropyridazine-3,6-dione. The benzofuran herbicide may be, for example, ethofumesate or 2,3-dihydro-3,3-dimethyl-benzofuran-5-yl ethanesulphonate. The arsenic herbicide generally comprises a salt, e.g. the mono- or di-

sodium salt of methane arsonic acid or cacodylic acid. Other herbicides which may be used include 1,2-dimethyl-3,5-diphenyl-pyrazolium ion, ethyl N-benzoyl-N-(3,4-dichlorophenyl)alanine, N-isobutyl-2-oxo-1-imidazolidine-carboxamide, aminotriazole,2,3-dichloro-1,4-naphthoquinone, 4-amino-3,5,6-trichloropicolinic acid, N,N-dimethyl-2,2-diphenylacetamide, 2,6-dinitro-N,N-dipropyl-4-trifluoromethyl-aniline, N-butyl-N-ethyl-2,6-dinitro-4-trifluoromethylaniline, S,S,S-tributyl phosphorotrithioate, 2-ethoxy-2,3-dihydro-3,3-dimethyl-5-benzofuranyl methylsulphonate, 4-chloro-2-oxobenzothiazolin-3-yl acetic acid, 3-isopropyl-2,1,3-benzothiadiazinon-(4)-2,2-dioxide, 3,5-dibromo-4-hydroxybenzaldehyde, 2,4-dinitrophenyloxime, methyl 2-chloro-3-(4-chlorophenyl)propionate, 2-chloroethyltrimethylammonium chloride, 4-methylsulphonyloxy-2-butynyl m-chlorocarbanilate, isopropyl 2-(N-benzoyl-3-chloro-4-fluoroanilino)propionate, methyl 2-(N-benzoyl-3-chloro-4-fluoroanilino)propionate, 2-chloro-N-(1,3-dioxolan-2-ylmethyl)-2',6'-dimethylacetanilide, 2-chloro-1-(3-ethoxy-4-nitrophenoxy)-4-trifluoromethylbenzene, methyl 2-(4-[2',4'-dichlorophenoxy]phenoxy)propionate, isobutyl 2-(4-[4'-chlorophenoxy]phenoxy)propionate, 1,1,1-trifluoro-2'-methyl-4'-(phenylsulphonyl)methane sulphonanilide, 4-chloro-5-methylamino-2-(3-trifluoromethylphenyl)-3(2H)-pyridazinone, 5-(2-chloro-4-trifluoromethylphenoxy)-2-nitrobenzoic acid, 1-methyl-3-phenyl-5-(3-trifluoromethylphenyl)-4-pyridone, 4-(methylsulphonyl)-2,6-dinitro-N,N-dipropylaniline, 4-(2,4-dichlorophenoxy)-2-methoxy-1-nitrobenzene, N-(1-ethylpropyl)-3,4-dimethyl-2,6-dinitroaniline, 2',4'-dimethyl-5'-(trifluoromethanesulphonamido)acetanilide, dimethyl 2,3,5,6-tetrachloroterephthalate, N-cyclopropylmethyl-2,6-dinitro-N-propyl-4-trifluoromethylaniline, N-(2-chloroethyl)-2,6-dinitro-N-propyl-4-trifluoromethylaniline, $N^1$,$N^1$-diethyl-2,6-dinitro-4-trifluoromethyl-m-phenylenediamine, $N^1$,$N^1$-dipropyl-2,6-dinitro-4-trifluoromethyl-m-phenylenediamine, N-sec-butyl-4-tert-butyl-2,6-dinitroaniline, 2-(3,4-dichlorophenyl)-4-methyl-1,2,4-oxadiazolidine-3,5-dione, 4-(dipropylamino)-3,5-dinitrobenzenesulphonamide, 1-(3-trifluoromethylphenyl)-3-chloro-4-chloromethyl-2-pyrrolidone, 2-(1-allyloxyaminobutylidine)-5,5-dimethyl-4-methoxycarbonylcyclohexane-1,3-dione, 2-(N-ethoxybutyrimidoyl)-5-(2-ethylthiopropyl)-3-hydroxy-2-cyclohexen-1-one, or 2-(1-(2,5-dimethylphenyl)ethylsulphonyl) pyridine N-oxide.

The compounds may also be employed in association with a herbicidal antidote (a substance having the property of improving the safety of a herbicide to a crop), e.g. N,N-diallyl-2,2-dichloroacetamide, 4'-chloro-2-(hydroxyimino)acetanilide, 1,8-naphthalic anhydride, α-(cyanomethoximino)-benzeneacetonitrile or 2,2-dimethyl-3-dichloroacetyloxazolidine. Although the antidote may be applied in admixture with active compound, it is preferably applied separately, and especially as a treatment for crop seeds. The ratio by weight of herbicide to antidote is preferably from 1:4 to 4:1.

The present compound may be used in admixture or sequence with another fungicide, particularly another potato fungicide. The other

fungicide may be for instance one or more of maneb (polymeric manganese ethylenebisdithiocarbamate), zineb (zinc ethylenebisdithiocarbamate), mancozeb (which can be regarded as a mixture of maneb and zineb), thiram (tetramethylthiuram disulphide), ditalimfos (O,O-diethyl phthalimidophosphonothioate), tridemorph (2,6-dimethyl-4-tridecylmorpholine), fluotrimazole (1-[diphenyl-(3-trifluoromethylphenyl)methyl]-1,2,4-triazole), ethirimol (5-butyl-2-ethylamino-4-hydroxy-6-methylpyrimidine), triforine (1,4-di[2,2,2-trichloro-1-formamidoethyl]piperazine), pyracarbolid (3,4-dihydro-6-methylpyran-5-carboxanilide), zinebethylene thiuramdisulphide adduct, carbendazim (methyl benzimidazol-2-ylcarbamate), captafol (3a,4,7,7a-tetrahydro-N-[1,1,2,2-tetrachloroethyanesulphenyl] phthalimide), thiophanate (1,2-di[3-ethoxycarbonyl-2-thioureido] benzene), proprineb (polymeric zinc propylenebisdithiocarbamate) oxycarboxin (2,3-dihydro-6-methyl-5-phenylcarbamoyl-1,4-oxathiin 4,4-dioxide), quintozene (pentachloronitrobenzene), benomyl (methyl 1-[butylcarbamoyl]benzimidazol-2-ylcarbamate), triadimefon and benadanil (2-iodobenzanilide).

The present compound may be used in admixture or sequence with an insecticide. The insecticide may be for instance one or more of bendiocarb, demeton-S-methyl (S-2-ethylthioethyl O,O-dimethyl phosphorothioate), dimethoate (O,O-dimethyl S-methylcarbamoylmethyl phosphorodithioate), formothion (S-[N-formyl-N-methylcarbamoylmethyl] O,O-dimethyl phosphorodithioate), oxydemeton-methyl (S-2-ethylsulphinylethyl O,O-dimethyl phosphorothioate), pirimicarb

(2-dimethylamino-5,6-dimethylpyrimidin-4-yl dimethylcarbamate), thiometon (S-2-ethylthioethyl 0,0-dimethyl phosphorodithioate), BHC (benzene hexachloride), aldrin (1,2,3,4,10,10-hexachloro-1,4a,4,5,8,8a-hexahydro-exo-1,4-endo-5,8-dimethanonaphthalene), fenitrothion (0,0-dimethyl 0-4-nitro-m-tolyl phosphorothioate), omethoate (0,0-dimethyl S-methylcarbamoylmethyl phosphorothioate), pirimiphos-methyl (0-2-diethylamino-6-methylpyrimidin-4-yl 0,0-dimethyl phosphorothioate) and DDT (1,1,1-trichloro-2,2-di[chlorophenyl]ethane).

The ratio of the present compound to the other pesticide or plant growth regulant may vary over a wide range according to the particular compounds involved and the intended use. In general the ratio of present compound to other pesticide or plant growth regulant lies in the range 1:0.1 to 1:15.

The present compounds may be in admixture with non-phytotoxic oils, e.g. Agri-Oil Plus, Sun Oil 11E or Fyzol E.

The compounds may be in admixture with fertilizers.

The compounds and compositions of the invention are active against a range of fungal diseases, particularly those of plants, such as *Phytophthora infestans* (potato blight), *Plasmopara viticola* (vine downy mildew), *Botrytis fabae* (chocolate spot of beans), *Erysiphe cichoraceearum* (powdery mildew of cucumber), *Puccinia striiformis* (wheat yellow rust) and *Uromyces phaseoli* (rust of french bean), and the damping off organisms *Pythium ultimum* and *Rhizoctonia solani*.

As mentioned above, the present invention provides a method of controlling or preventing fungal growth which comprises applying to a locus infested or liable to be infested by fungus a compound or composition according to the invention.

The locus infected or liable to be infected may be plants, animals, the soil, aquatic areas, fabrics, textiles, paper, wood and the like.

Preferably the compound or composition is employed against fungal diseases of plants. The plants may be growing or may be seeds, using 'seeds' in the wider sense to include tubers etc. The compound or composition can be applied directly to the plants, or can be applied to the medium in which they grow and be taken up by the plants and distributed within the plants, i.e. the compounds show systemic activity.

The compounds are applied to a locus infested or liable to be infested with fungus at a rate for example of 0.2 to 5 kg per hectare. For use as a seed dressing, the compounds may be applied in the form of a dust with a solid carrier for instance at the rate of 1-4 e.g. 2 oz of dust per bushel of seed or may be applied in the form of a liquid with a liquid carrier for instance at a rate of 0.75-3 fluid oz of liquid per bushel of seed.

The compounds and compositions according to the invention also possess herbicidal activity, especially against weeds growing in crops such as cotton (Gossypium sp), potato (Solanum tuberosum), peanuts (Arachis hypogaea), maize (Zea mays), sunflower (Helianthus

annus), rice (Oryza sativa), sorghum (Sorghum vulgare), soya beans (Soja max), millets, e.g. foxtail millet (Setaria italica), pearl millet (Pennisetum typhoides) and common millet (Panicum miliaeceum), oats (Avena sativa), peas (Pisum sativum), navy beans (Phaseolus vulgaris), wheat (Triticum aestivum) and barley (Hordeum vulgare).

Particular weeds which may be mentioned include broad leaved, rhizomatous and grass weeds, e.g. purslane (Portulaca oleracea), ground cherry (Physalis longiflora), jimsonweed (Datura stramomium), pigweeds (Amaranthus spp), mayweeds (Triplevrospermum maritimum), and (Natricaria matricariodes), chickweed (Stellaria media), fat hen (Chenopodium album), charlock (Sinapis arrensis), (also known as Brassica kaber), blackgrass (Alopecurus myosuroides), canary grass (Phalaris arundinace), sterile brome (Bromus sterilis), annual meadowgrass (Poa annua), yellow nutsedge (Cyperus resculentus), and purple nutsedge (Cyperus rotundus).

The compounds and compositions may be applied to the area, e.g. land, soil or water, infested or liable to be infested with the weeds either before or after emergence of the weeds and/or the crop.

The present compounds are usually employed for herbicidal use at a rate of from 0.5 to 8 kg, e.g. 1 to 4 kg per hectare.

We prefer to use compounds of formula I in which X is -CN and Y is a cation as fungicides. Of the compounds of formula I in which X and Y together form a group of formula II we prefer to use those

in which $R^6$ is -COOH or -COSH or esters thereof as herbicides and those in which $R^6$ has its other significances as fungicides. In particular we prefer to use compounds of formulae Ic and Id as herbicides and compounds of formula Ie as fungicides.

The invention is illustrated by, but not limited to, the following Examples in which temperatures are in $^{o}C$, Me represents methyl, Et represents ethyl and Ph represents phenyl.

Example 1

(a) Amino-(decylamino)-methylenepropanedinitrile

Decylammonium tricyanomethanide (8g) was heated under reflux with xylene (50 ml) for 4 hours. The solid was filtered off and recrystallised from ethanol to give the yellowish, waxy title product (4.4g, 55%), m.p. 98-100$^{o}$.

| | | | |
|---|---|---|---|
| Found: | C, 67.7; | H, 9.75; | N, 22.55 |
| $C_{14}H_{24}N_4$ requires: | C, 67.7; | H, 9.7; | N, 22.6%. |

Similarly prepared was:

(b) Amino-(octadecylamino)-methylenepropanedinitrile m.p. 109-11$^{o}$.

Example 2

(a) Amino-(1-piperidinyl)-methylenepropanedinitrile

Piperidinium tricyanomethanide (32g) was heated under reflux in piperidine (200 ml) for 4 hours. The solution was cooled and poured into a mixture of ice and water (800 ml) to precipitate the title product (19.6g, 61%), m.p. 165-6$^{o}$.

Similarly prepared was:-

(a) Amino-(4-morpholinyl)-methylenepropanedinitrile m.p. 205-10$^{o}$.

The process was repeated, giving satisfactory results, using xylene, 2-methoxyethanol and water respectively in place of the morpholine as solvent.

Example 3

(a) Amino-(1,6-hexanediylamino)-methylenepropanedinitrile

1,6-Hexanediylammonium tricyanomethanide was heated in 1,6-hexanediylamine (50 ml), under reflux, for 10 hours. The mixture was poured into 3M-hydrochloric acid (150 ml) and ice (150g), and the precipitate was recrystallised from ethanol to give the title product (13g, 68% overall), m.p. 164-5°.

Similarly prepared were:-

(b) Amino-(N-butyl-N-ethylamino)-methylenepropanedinitrile m.p. 107-10°.

(c) Amino-(dipropylamino)-methylenepropanedinitrile m.p. 123-5°.

Example 4

(a) Amino-(3,4-dichlorophenylamino)-methylenepropanedinitrile

3,4-Dichlorophenylammonium tricyanomethanide was heated under reflux in water (10 ml/g) for 4 hours to give a precipitate of the title product (71% yield). Recrystallisation from 2-methoxyethanol gave the pure title product (58%), m.p. 230° decomp.

| | | | |
|---|---|---|---|
| Found: | C, 47.15; | H, 2.8; | N, 21.65 |
| $C_{10}H_6Cl_2N_4$ requires: | C, 47.45; | H, 2.4; | N, 22.15% |

(b) Amino-(2,4-dibromophenylamino)-methylenepropanedinitrile

2,4-Dibromobenzenamine (5g, 0.02M), potassium tricyanomethanide (2.8g, 0.022M), c-hydrochloric acid (1.8 ml, 0.02M approx), and water (20 ml) were heated under reflux for 12 hours. The mixture was cooled and the precipitate (3.5g, 51%) was recrystallised from 2-methoxyethanol to give the title product (1.5g, 22%), m.p. 255° decomp.

Found: C, 35.15; H, 2.1; N, 16.7

$C_{10}H_6Br_2N_4$ requires: C, 35.1; H, 1.75; N, 16.4%

The compounds listed in Table I were obtained by the methods of this Example.

Table I

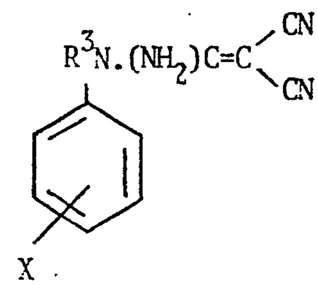

| | $R^3$ | X | m.p. ° |
|---|---|---|---|
| 1 | H | 2,5-diCl | 200-203 |
| 2 | H | 2-Br | 189-94 |
| 3 | H | 4-Br | 270 decomp |
| 4 | H | 2-Cl | 203-5 |
| 5 | H | 3-Cl | 209-11 |
| 6 | H | 4-Cl | 208-10 |
| 7 | H | 4-F | 190-92 |
| 8 | H | 2-I | 210-12 |
| 9 | H | 4-I | 278 decomp |
| 10 | H | $4-NO_2$ | 260 decomp |
| 11 | H | $3-NH_2$ | 250 decomp |
| 12 | H | $3-CF_3$ | 198-200 |

| | $R^3$ | X | m.p. |
|---|---|---|---|
| 13 | H | 4-Br-3-Me | 202-4 |
| 14 | H | 2-Cl-6-Me | 206-7 |
| 15 | H | 3-Cl-2-Me | 244-5 |
| 16 | H | 4-Cl-2-Me | 265-7 |
| 17 | H | 5-Cl-2-Me | 215-7 |
| 18 | H | 4-Me | 242-5 decomp |
| 19 | H | 2-OMe | 177-9 |
| 20 | H | 3-OMe | 196-7 |
| 21 | H | 4-OMe | 244-5 decomp |
| 22 | H | 3,5-di-Me | 265-70 |
| 23 | H | $2-Me_2CH$ | 216-7 |
| 24 | H | 2,5-diOEt | 190-91 |
| 25 | H | 4-OPh | 208-10 |
| 26 | $C_2H_5$ | H | 151-3 |

Example 5

Potassium 4-(1-amino-2,2-dicyanoethenylamino)-benzenesulphonate hydrate

4-Aminobenzenesulphonic acid (17.3g, 0.1M) and potassium tricyanomethanide (14g, 0.11M) were heated under reflux in water (100 ml) for 8 hours. The solution was cooled to precipitate the title product (22.4g, 68%), m.p. 250$^{\circ}$ decomp.

Example 6

Amino-(1-piperazinyl)-methylenepropanedinitrile

Piperazine (15g, 0.17M), potassium tricyanomethanide (14g, 0.11M), c-hydrochloric acid (9 ml, 0.1M approx), and water (30 ml) were heated under reflux for 14 hours. The solution was left at room temperature for 4 days, during which time there crystallised out the title product (13.5g, 70%), m.p. 180-84°.

| | | | |
|---|---|---|---|
| Found: | C, 54.2; | H, 6.3; | N, 39.8 |
| $C_8H_{11}N_5$ requires: | C, 54.2; | H, 6.25; | N, 39.5% |

Example 7

(a) Amino-(2-amino-3,5-dibromo-4-chlorophenylamino)-methylene-dinitrile

3,5-Dibromo-4-chloro-1,2-benzenediamine (15g, 0.05M), potassium tricyanomethanide (7g, 0.055M), conc. HCl (4.5 ml), and water (50 ml) were heated under reflux for 48 hours. The precipitate was stirred with warm 1M-sodium hydroxide and insoluble material was filtered off. The filtrate was neutralised to pH 7 with hydrochloric acid. The precipitate was recrystallised from 2-methoxyethanol/water to give the title product (7g, 36%), m.p. 200° decomp.

Similar reactions to that above, using the appropriate benzenediamines, gave the following compounds, after heating for 14-36 hours:

(b) Amino-(2-amino-3,4,5-trichlorophenylamino)-methylenepropanedinitrile, m.p. 180° decomp.

(c) Amino-(2-amino-3,5-dibromophenylamino)-methylenepropanedinitrile m.p. 258° decomp.

(d) Amino-(2-amino-4,5-dichlorophenylamino)-methylenepropanedinitrile m.p. 207° decomp.

(e) Amino-(2-amino-3,5-diiodophenylamino)-methylenepropanedinitrile m.p. 250° decomp.

(f) Amino-(2-aminophenylamino)-methylenepropanedinitrile m.p. 285-7°.

(g) Amino-(2-amino-4,5-dimethylphenylamino)-methylenepropanedinitrile m.p. 195° decomp.

(h) Amino-(2-amino-3-chloro-5-iodophenylamino)-methylenepropanedinitrile, m.p. 239-40° decomp.

Example 8

2-[(1-Amino-2,2-dicyanoethenyl)-amino]-5-chlorobenzoic acid

2-Amino-5-chlorobenzoic acid (17.2g, 0.1M), potassium tricyanomethanide (14g, 0.11M), c-hydrochloric acid (9 ml, 0.1M approx) and water (100 ml) were heated under reflux for 12 hours. The precipitate (20.8g, 79%), decomp 260°, was dissolved in 1M-sodium hydroxide. Acidification with hydrochloric acid gave a precipitate of the title product (16.6g, 63%), decomp 260°.

Example 9

(a) Methylamino-(1-piperidinyl)-methylenepropanedinitrile

Amino-(1-piperidinyl)-methylenepropanedinitrile (12.5g, 0.07M),

iodomethane (25g, 0.18M), potassium carbonate anhyd. (14.3g, 0.105M), and acetone (100 ml) were heated under reflux for 20 hours. The mixture was filtered hot and then reduced in volume to remove excess iodomethane. The solution was poured into water to give an oil which crystallised after a few minutes. The solid was recrystallised from ethanol to give the title product (5g, 37%) mp 155-

(b) <u>Amino-(N-methyl-3-chlorophenylamino)-methylenepropanedinitrile</u>

was prepared similarly, except that the reaction mixture was not filtered but poured straight into water. Recrystallisation of the precipitate from 2-methoxyethanol gave the title product (17%), m.p. 236-7°.

<u>Example 10</u>

(a) <u>3-Amino-3-(2-bromophenylamino)-2-cyanopropenamide</u>

The 2-bromophenylammonium salt of dicyanoacetamide (16.6g, 0.06M) was heated under reflux in water (60 ml) for 8 hours to give a precipitate of the title product (12.7g, 77%). Recrystallisation from 2-methoxyethanol/water gave the title product (9.8g, 59%), m.p. 210-11°.

Heating the appropriate salts in water similarly gave the following compounds;

(b) <u>3-Amino-3-(2-iodophenylamino)-2-cyanopropenamide</u> m.p. 124-5°.

(c) <u>3-Amino-3-(4-chloro-2-methylphenylamino)-2-cyanopropenamide</u> m.p. 224-6°.



BAD ORIGINAL

Example 11

(a) 3-Amino-3-(2,4-dichlorophenylamino)-2-cyanopropenamide

2,4-Dichlorobenzenamine, (16.2g, 0.1M), potassium salt of dicyanoacetamide (16g, 0.11M), c-hydrochloric acid (9 ml, 0.1M approx), and water (100 ml) were heated under reflux for 20 hours to give a precipitate of the title product (21.5g, 79%). Recrystallisation from 2-methoxyethanol gave the pure product (10g, 37%), m.p. 228-30°.

(b) 3-Amino-3-(4-bromophenylamino)-2-cyanopropenamide

4-Bromoaniline (86g, 0.5M), the potassium salt of dicyanoacetamide (80g, 0.54M), concentrated hydrochloric acid (45 ml, 0.5M) and water (350 ml) were heated under reflux for 10 hours and then cooled to room temperature. The product was filtered off and recrystallised from 2-methoxyethanol. (83g, 59%) m.p. 203-4°.

Found: C, 42.6; H, 3.4; N, 20.4

$C_{10}H_9BrN_4O$ requires: C, 42.7; H, 3.25; N, 19.95%

(c) 3-Amino-3-(4-chlorophenylamino)-2-cyanopropenamide

4-Chloraniline (63.8g, 0.5M), the potassium salt of dicyanoacetamide (80g, 0.54M), concentrated hydrochloric acid (45 ml, ~0.5M) and water (350 ml) were heated under reflux for 8 hours. The mixture was cooled and the product was filtered off and recrystallised from 2-methoxyethanol. (62g, 52%), m.p. 218-9°C.

Found: C, 50.35; H, 3.65; N, 24.05

$C_{10}H_9ClN_4O$ requires: C, 50.75; H, 3.85; N, 23.7%

(d) 3-Amino-3-(4-chloro-3-methylphenylamino)-2-cyanopropenamide

The product of Example 27 (52.3g), potassium salt of dicyanoacetamide (59g), concentrated hydrochloric acid (33.5 ml) and water (270 ml) were heated under reflux for 20 hours. The mixture was cooled and the product was filtered off and recrystallised from 2-methoxyethanol, (70g, 75%), m.p. 180-81°.

| | | | |
|---|---|---|---|
| Found: | C, 52.95; | H, 4.65; | N, 21.9 |
| $C_{11}H_{11}ClN_4O$ requires: | C, 52.7; | H, 4.4; | N, 22.35% |

(e) 3-Amino-3-(1,2,3,4-tetrahydro-1-quinolinyl)-2-cyanopropenamide m.p. 177-8° was prepared similarly.

Similar reactions, using appropriate benzenamines, gave the compounds listed in Table II.

Table II

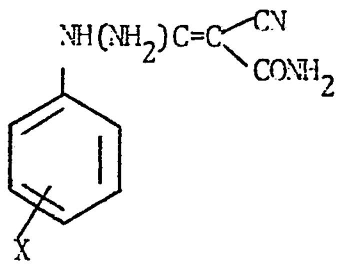

| | X | m.p. ° |
|---|---|---|
| 1 | 3,4-diCl* | 215 |
| 2 | 3,5-diCl | 233-4 |
| 3 | 2-Cl | 212-3 |
| 4 | 3-Cl | 217-9 |
| 5 | 4-F | 245-6 |
| 6 | 4-I | 215 decomp |
| 7 | $3-NO_2$ | 233-5 |
| 8 | H | 194-5 |
| 9 | $3-CF_3$ | 112-6 |
| 10 | 4-Br-3-Me | 193-4 |
| 11 | 3-Cl-2-Me | 205-7 |
| 12 | 3-Me | 160-63 |
| 13 | 4-Me | 197-8 |
| 14 | 2-MeO | 181-2 |
| 15 | 2,4-diMe | 181-2 |
| 16 | 2,5-diMe | 177-9 |

| | X | m.p. ° |
|---|---|---|
| 17 | 2,6-diMe | 182-4 |
| 18 | 2-$C_2H_5$ | 164-5 |
| 19 | 2-$Me_2CH$ | 215-6 |
| 20 | 2,5-di-EtO | 183-4 |

* This compound was also prepared by heating amino-(3,4-dichlorophenylamino)-methylenepropanedinitrile in 2M aqueous sodium hydroxide for 4 hours.

The compounds listed in Table III were prepared by similar methods using the potassium salt of N-methyl dicyanoacetamide in place of the salt of dicyanoacetamide.

Table III

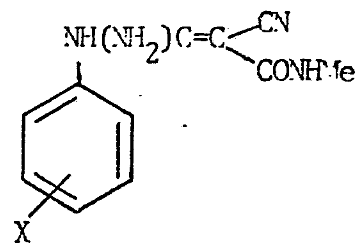

| | X | m.p. ° |
|---|---|---|
| 1 | 3,5-diCl | 203-4 |
| 2 | 4-Br | 191-2 |
| 3 | 3-Cl | 163-4 |
| 4 | 4-Cl | 185 |
| 5 | 4-F | 185-7 |
| 6 | 4-I | 201-3 |
| 7 | $3\text{-}NO_2$ | 260 |
| 8 | $4\text{-}NO_2$ | 214-5 |
| 9 | H | 99-100 |
| 10 | 4-Br-3-Me | 183-5 |
| 11 | 3-Me | 75-7 |
| 12 | 4-Me | 91-3 |
| 13 | 4-MeO | 185-8 |
| 14 | $2\text{-}C_2H_5$ | 99-104 |

Example 12

Potassium 4-[(1,3-diamino-2-cyano-3-oxopropenyl)-amino]-benzenesulphonate hydrate

4-Aminobenzenesulphonic acid (17.3g, 0.1M), potassium salt of dicyanoacetamide (16g, 0.11M), and water (100 ml) were heated under reflux for 8 hours. The solution was cooled to precipitate the title product (69%), m.p. 270$^{o}$ decomp.

Example 13

Potassium 4-(1-amino-3-methylamino-2-cyano-3-oxopropenylamino)-benzenesulphonate dihydrate

4-Aminobenzenesulphonic acid (8.7g, 0.05M), potassium salt of *N*-methyldicyanoacetamide (9g, 0.055M), and water (50 ml), were heated under reflux for 40 hours. The solution was cooled to precipitate the title product (7.6g, 41%), m.p. 200$^{o}$ decomp.

Example 14

3-Amino-3-(3-chlorophenylamino)-2-cyano-N-ethylpropenamide

3-Chlorobenzenamine (6g, 0.037M), potassium salt of *N*-ethyldicyanoacetamide (7g, 0.043M), *c*-hydrochloric acid (3.4 ml, 0.037M approx), and water (40 ml) were heated under reflux for 12 hours. The precipitate was recrystallised from ethanol to give the title product (2.3g, 24%), m.p. 131-3$^{o}$.

Also made by this method was 3-amino-3-(4-chlorophenylamino-)-2-cyano-N-ethylpropenamide, mp 141-3$^{o}$C.

Example 15

3-Amino-3-phenylamino-2-cyano-N,N-dimethylpropenamide

A solution of propanedinitrile (6.6g, 0.1M) and dimethylcarbamyl chloride (11g, 0.102M) in tetrahydrofuran was added, with stirring, to a solution of potassium hydroxide (11.2g, 0.2M) in water, over 15 min. The temperature of the mixture was kept below 30° by water cooling. At the end of the addition, the pH of the solution was 8. The solution was stirred for a further 2 hours and then evaporated to dryness to yield the potassium salt of N,N-dimethyldicyanoacetamide. The residue was washed with a little ethanol, dried and dissolved in water (50 ml). Benzenamine hydrochloride (10.4g, 0.08M) was added and the solution was heated under reflux for 14 hours and then cooled to room temperature. The precipitate (1.4g) was recrystallised from ethanol to give the title product (0.5g, 2.5% based on propanedinitrile), m.p. 134-5°.

Example 16

(a) 3-Amino-3-(3,4-dichlorophenylamino)-2-cyano-N-phenylpropenamide

3,4-Dichlorobenzenamine (8.1g, 0.05M), N-phenyldicyanoacetamide containing 20% of its triethylamine salt (10.25g, of mixture, equivalent to 0.05M of the acid), c-hydrochloric acid (1 ml, 0.01M approx, to neutralise the triethylamine), and water (50 ml) were heated under reflux for 4 hours. The precipitate (7.4g, 46%) was recrystallised from 2-methoxyethanol to give the title product (7.4g, 46%), m.p. 208-10°.

Similar reactions, using the appropriate benzenamines, gave the following compounds after heating for from 8 to 48 hours.

(b) 3-Amino-3-(4-bromophenylamino)-2-cyano-N-phenylpropenamide m.p. 185-7°.

(c) 3-Amino-3-(3-chlorophenylamino)-2-cyano-N-phenylpropenamide m.p. 135-6°.

(d) 3-Amino-3-(4-chlorophenylamino)-2-cyano-N-phenylpropenamide m.p. 194°.

(e) 3-Amino-3-(4-fluorophenylamino)-2-cyano-N-phenylpropenamide m.p. 174-6°.

(f) 3-Amino-3-(3-nitrophenylamino)-2-cyano-N-phenylpropenamide m.p. 168-70°.

(g) 3-Amino-3-(4-nitrophenylamino)-2-cyano-N-phenylpropenamide m.p. 214-5°.

(h) 3-Amino-3-phenylamino-2-cyano-N-phenylpropenamide m.p. 164°.

(i) 3-Amino-3-(3-methylphenylamino)-2-cyano-N-phenylpropenamide m.p. 139-41°.

(j) 3-Amino-2-cyano-3-(4-methoxyphenylamino)-N-phenylpropenamide m.p. 196-200°.

(k) 3-Amino-3-(2-ethylphenylamino)-2-cyano-N-phenylpropenamide mp 138-40°

Example 17

Ethyl 3-amino-2-cyano-3-(1-piperidinyl)-propenoate

The piperidine salt of ethyl dicyanoacetate (7.5g, 0.033M) was heated under reflux in piperidine (20 ml) for 24 hours. The solution was poured into a mixture of ice (70g) and c-hydrochloric

acid (25 ml) and the precipitate was recrystallised from ethanol to give the title product (1.8g, 25%), m.p. 104-6°.

Example 18

(a) Ethyl 3-amino-3-(3,4-dichlorophenylamino)-2-cyanopropenoate

3,4-Dichlorobenzenamine (16.2g, 0.1M), potassium salt of ethyl dicyanoacetate (19.4g, 0.11M) c-hydrochloric acid (9 ml, 0.1M approx), and water (100 ml) were heated under reflux for 6 hours. The precipitate (27g, 90%) was recrystallised from ethanol to give the title product (21.1g, 70%), m.p. 155-6°.

(b) Ethyl 3-amino-2-cyano-3-(N-methylphenylamino)propenoate

Potassium salt of ethyl dicyanoacetate (554g), N-methylaniline (315g), water (1470 ml), and concentrated hydrochloric acid (265 ml) were heated under reflux for 20 hours. The mixture was cooled and the precipitate was filtered off and washed with water. Recrystallisation from ethanol gave the title product (449g, 59%), m.p. 147-9°.

| | | | |
|---|---|---|---|
| Found: | C, 64.05; | H, 6.4; | N, 17.55 |
| $C_{13}H_{15}N_3O_2$ requires: | C, 63.65; | H, 6.15; | N, 17.15% |

(c) Isopropyl 3-amino-2-cyano-3-(N-methylphenylamino)-propenoate

(i) The product of Example 37 (18.1g, 0.095M), N-methyl aniline (9.7 ml, 9.57g, 0.0895M), water (47.5 ml), and concentrated hydrochloric acid (8.5 ml, 0.9M approx) were heated under reflux for 20 hours. The mixture was cooled to room temperature and the precipitate was recrystallised from isopropanol to give the title compound (14.5g, 62%), mp 147-9°C.

Found: C, 65.15; H, 6.45; N, 16.25

$C_{14}H_{17}N_3O_2$ Requires: C, 64.85; H, 6.6; N, 16.2%

(ii) The reaction described in Example 37 was repeated, but the reaction mixture was not cooled to $0^oC$, instead, N-methylaniline (21 ml, 20.7g, 0.194n) and concentrated hydrochloric acid (19 ml, 0.194n approx) were added, and the mixture was heated under reflux for 20 hours. The mixture was cooled to room temperature and the precipitate was recrystallised from isopropanol to give the title product (29.3g, 47% based on malononitrile). The i.r. and pmr spectra of the product were identical to those of the sample prepared by method (i) above.

The compounds listed in Table IV were prepared by the methods of this Example using appropriate starting materials.

Table IV

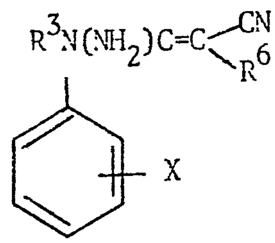

| | $R^6$ | $R^3$ | X | m.p. ° |
|---|---|---|---|---|
| 1 | $CO_2Et$ | H | 4-Br | 208-9 |
| 2 | $CO_2Et$ | H | 3-Cl | 135-7 |
| 3 | $CO_2Et$ | H | 4-F | 166-8 |
| 4 | $CO_2Et$ | H | $3-NO_2$ | 160 |
| 5 | $CO_2Et$ | H | $4-NO_2$ | 187-8 |
| 6 | $CO_2Et$ | H | H | 169-71 |
| 7 | $CO_2Et$ | H | $2-CO_2H$ | 220-22 |
| 8 | $CO_2Et$ | H | 4-Br-3-Me | 157-8 |
| 9 | $CO_2Et$ | H | 3-Me | 121-3 |
| 10 | $CO_2Et$ | H | 4-Me | 143-5 |
| 11 | $CO_2Et$ | H | 2-OMe | 201-2 |
| 12 | $CO_2Et$ | H | 4-OMe | 174-6 |
| 13 | $CO_2Et$ | H | $2-C_2H_5$ | 129-32 |
| 14 | $CO_2Et$ | Me | 3-Cl | 142-4 |
| 15 | $CO_2-nC_3H_7$ | Me | H | 149-50 |
| 16 | $CO_2-nC_4H_9$ | Me | H | 128-30 |

| | $R^6$ | $R^3$ | X | m.p. ° |
|---|---|---|---|---|
| 17 | $CO_2-CH_2CHMe_2$ | Me | H | 184-6 |
| 18 | $CH_3$<br>$CO_2-CHCH_2CH_3$ | Me | H | 133-5 |
| 19 | $CH_3$<br>$CO_2-CH_2CHCH_2CH_3$ | Me | H | 153-5 |
| 20 | $CO_2-CH_2CMe_3$ | Me | H | 179-81 |
| 21 | $CH_3$<br>$CO_2-CH-nC_5H_{11}$ | Me | H | 94-5 |
| 22 | $CO_2-nC_8H_{17}$ | Me | H | 118-20 |
| 23 | $CO_2-nC_{11}H_{23}$ | Me | H | 88-90 |

Example 19

Potassium 4-(1-amino-2-cyano-3-oxo-3-ethoxypropenylamino)-benzene-sulphonate hemihydrate

4-Aminobenzenesulphonic acid (8.7g, 0.05M), potassium salt of ethyl dicyanoacetate (9g, 0.051M), and water (50 ml) were heated under reflux for 36 hours. The solution was evaporated to dryness and the residue was recrystallised from a small amount of water to give the title product (6g, 34%), m.p. 260° decomp.

Example 20

Ethyl 3-amino-3-(N-methyl-3,4-dichlorophenylamino)-2-cyano-propenoate

Ethyl 3-amino-2-cyano-3-(3,4-dichlorophenylamino)-propenoate

(14.3g, 0.048M), iodomethane (16 ml, 14g, 0.1M), potassium carbonate, anhyd. (7.5g, 0.54M), and acetone (100 ml) were heated under reflux, with stirring, for 14 hours. The mixture was cooled and filtered, and the filtrate was evaporated to dryness. The residue was recrystallised from ethanol to give the title product (2g, 13%), m.p. 198-9$^{o}$.

Example 21

Phenyl 3-Amino-3-phenylamino-2-cyanopropenoate

A solution of propanedinitrile (6.6g, 0.1M) in phenyl chloroformate (13 ml, approx 16g, 0.103M) was added slowly, with stirring, to a solution of potassium hydroxide (11.2g, 0.2M) in water (25 ml) with ice cooling to keep the temperature of the mixture below 40$^{o}$. The mixture was stirred at 30-40$^{o}$ for a further 15 minutes and then cooled to 0$^{o}$. A small amount of precipitate was found to be diphenyl carbonate. The filtrate was evaporated to dryness at reduced pressure. The residue was washed with ether and extracted with acetone (50 ml). The extracts were evaporated and the residue was washed with ether and dried. The potassium salt of phenyl dicyanoacetate (10.5g, 0.0-7M, 47%) thus obtained was heated under reflux with aniline hydrochloride (6.1g, 0.047M) in water (58 ml) for 24 hours. The sticky precipitate was triturated with ethanol to give the title compound as a blue-grey powder (4.3g, 15% from propanedinitrile). Recrystallisation from dimethylformamide gave the pure product (2g, 7%), m.p. 214-5$^{o}$.

Example 22

(a) S-Ethyl 3-amino-3-(3,4-dichlorophenylamino)-2-cyanopropenethioate

3,4-Dichlorobenzeneamine (5.4g, 0.033M), potassium salt of S-ethyl dicyanoethanethioate (7g, 0.036M), c-hydrochloric acid (3 ml, 0.033M approx), and water (35 ml) were heated under reflux for 20 hours. The oil which separated was triturated with 2-propanol to give the title product (6.8g, 63%), m.p. 142-5°.

Similar reactions, using the appropriate benzenamines, gave the compounds listed in Table V.

Table V

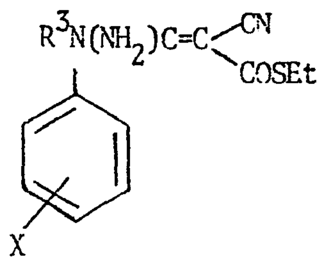

| | $R^3$ | X | m.p. ° |
|---|---|---|---|
| 1 | H | 4-Br | 154-6 |
| 2 | H | 3-Cl | 107-16 decomp |
| 3 | H | 4-Cl | 145-7 |
| 4 | H | 4-I | 210-15 |
| 5 | H | $3-NO_2$ | 177-80 |
| 6 | H | H | 128-9 |
| 7 | H | $2-NH_2$ | 135-7 |

| | $R^3$ | X | m.p. ° |
|---|---|---|---|
| 8 | H | 3-Me | 120-22 |
| 9 | H | 4-Me | 118-20 |
| 10 | H | 2-OMe | 153-5 |
| 11 | H | 4-OMe | 160-63 |
| 12 | Me | H | 137-9 |

Example 23

(Intermediates for Example 24)

(a) 4-Chlorophenylamino-(methylthio)-methylenepropanedinitrile

4-Chlorobenzeneamine (64g, 0.5M), bis-methylthiomethylene-propanedinitrile (85g, 0.5M), and ethanol (400 ml) were heated under reflux for 6 hours. The solution was cooled and the precipitate was recrystallised from ethanol to give the title product (65g, 52%), m.p. 164-6°.

Similar reactions, using appropriate benzeneamines and 1,1-bis-methylthioethylenes in ethanol, or, in the case of the esters using ethanol or the related alcohol, gave the compounds listed in Table VI.

Table VI

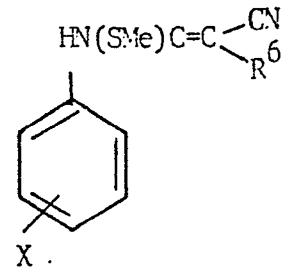

| | $R^6$ | X | m.p. ° |
|---|---|---|---|
| 1 | CN | 3,4-diCl | 193-5 |
| 2 | CN | 4-Br | 201-2 |
| 3 | CN | 3-Cl | 161-3 |
| 4 | CN | 4-Cl | 164-6 |
| 5 | CN | H | 174-5 |
| 6 | CN | 3-Me | 132-4 |
| 7 | CN | 4-Me | 164-5 |
| 8 | $CONH_2$ | 3,4-diCl | 190-92 |
| 9 | $CONH_2$ | 4-Br | 212-5 |
| 10 | $CONH_2$ | 3-Cl | 174-6 |
| 11 | $CONH_2$ | 4-Cl | 200-203 |
| 12 | $CONH_2$ | $4-NO_2$ | 214-5 |
| 13 | $CONH_2$ | H | 151-3 |
| 14 | $CONH_2$ | 3-Me | 143-4 |
| 15 | $CONH_2$ | 4-Me | 183-5 |
| 16 | $CO_2Me$ | 3,4-diCl | 140-42 |

| | $R^6$ | X | m.p. ° |
|---|---|---|---|
| 17 | $CO_2Me$ | 4-Br | 140-42 |
| 18 | $CO_2Me$ | 3-Cl | 105-7 |
| 19 | $CO_2Me$ | 4-Cl | 134-7 |
| 20 | $CO_2Me$ | $3-NO_2$ | 183-5 |
| 21 | $CO_2Me$ | $3-CF_3$ | 143-6 |
| 22 | $CO_2Me$ | 3-Me | 93-4 |
| 23 | $CO_2Me$ | 4-Me | 93-5 |
| 24 | $CO_2Et$ | 3,4-diCl | 115-9 |
| 25 | $CO_2Et$ | 3,5-diCl | 140 |
| 26 | $CO_2Et$ | 4-Br | 130-31 |
| 27 | $CO_2Et$ | 3-Cl | 118-20 |
| 28 | $CO_2Et$ | 4-Cl | 105-6 |
| 29 | $CO_2Et$ | 4-F | 102-4 |
| 30 | $CO_2Et$ | $3-NO_2$ | 150-52 |
| 31 | $CO_2Et$ | H | 77-9 |
| 32 | $CO_2Et$ | $3-CF_3$ | 122-3 |
| 33 | $CO_2Et$ | 3-Me | 107-9 |
| 34 | $CO_2Et$ | 4-Me | 75-6 |
| 35 | $CO_2Et$ | 3-OMe | 120-22 |
| 36 | $CO_2Et$ | 4-OMe | 85-6 |
| 37 | $CO_2Et$ | $4-NMe_2$ | 139-42 |
| 38 | $CO_2CH_2CH_2CH_3$ | 4-Cl | 64-6 |
| 39 | $CO_2CHMe_2$ | H | 90 |
| 40 | $CO_2CMe_3$ | H | 92-5 |

Example 24

(a) Methylamino-(4-chlorophenylamino)-methylenepropanedinitrile

4-Chlorophenylamino-(methylthio)-methylenepropanedinitrile (7.5g, 0.03M), methylamine (4.8 ml of a 27.5% w/v solution in ethanol = 1.3g, 0.042M), and ethanol (25 ml) were heated under reflux for 6 hours. By that time p.m.r spectroscopy showed that no starting material (recognisable from absorption by its SMe group) remained. The solution was cooled and the precipitate was recrystallised from ethanol to give the title product (4.9g, 70%), m.p. 188-90°.

Similar reactions in ethanol (or, in the case of esters, in the appropriate alcohol), using appropriate arylamino-(methylthio)-ethylenes and either methylamine (27.5% w/v ethanol), dimethylamine (25% w/v in water), or ethylamine (70% w/v in water), or aqueous ammonia, or another appropriate amine, (ammonia generally needed longer reaction times (10-48 hours)), gave the compounds listed in Table VII.

Table VII

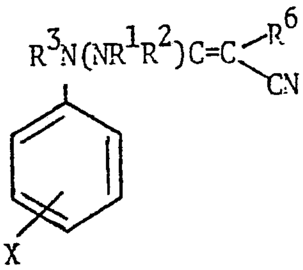

| | $R^6$ | $R^3$ | $R^1$ | $R^2$ | X | m.p. ° |
|---|---|---|---|---|---|---|
| 1 | CN | H | Me | H | 3,4-diCl | 215-7 |
| 2 | CN | H | Me | H | 4-Br | 180-81 |
| 3 | CN | H | Me | H | H | 168-70 |
| 4 | CN | H | Me | H | 3-Me | 175-7 |
| 5 | CN | H | Me | H | 4-Me | 200-203 |
| 6 | CN | H | Me | Me | 3,4-diCl | 210-12 |
| 7 | CN | H | Me | Me | 4-Br | 120-21 |
| 8 | CN | H | Me | Me | 3-Cl | 186-8 |
| 9 | CN | H | Me | Me | H | 186-8 |
| 10 | CN | H | Me | Me | 3-Me | 146-8 |
| 11 | CN | H | Me | Me | 4-Me | 180-82 |
| 12 | CN | H | Et | H | 3,4-diCl | 183-6 |
| 13 | CN | H | Et | H | 4-Br | 194-6 |
| 14 | CN | H | Et | H | 3-Cl | 163-5 |
| 15 | CN | H | Et | H | 4-Cl | 160-63 |
| 16 | CN | H | Et | H | H | 140-43 |
| 17 | CN | H | Et | H | 3-Me | 158-9 |

| | $R^6$ | $R^3$ | $R^1$ | $R^2$ | X | m.p. ° |
|---|---|---|---|---|---|---|
| 18 | CN | H | Et | H | 4-Me | 169-71 |
| 19 | CN | Me | Me | H | H | 109-10 |
| 20 | CN | Me | Me | Me | H | 133 |
| 21 | CN | Me | Et | H | H | 66-8 |
| 22 | $CONH_2$ | H | H | H | 4-$NO_2$ | 264-6 |
| 23 | $CONH_2$ | H | Me | H | 3,4-diCl | 187-9 |
| 24 | $CONH_2$ | H | Me | H | 4-Br | 215-7 |
| 25 | $CONH_2$ | H | Me | H | 4-Cl | 205-7 |
| 26 | $CONH_2$ | H | Me | H | 4-$NO_2$ | 210-11 |
| 27 | $CONH_2$ | H | Me | H | H | 207-9 |
| 28 | $CONH_2$ | H | Me | H | 3-Me | 182-4 |
| 29 | $CONH_2$ | H | Me | H | 4-Me | 210-11 |
| 30 | $CONH_2$ | H | Me | Me | 3,4-diCl | 185-7 |
| 31 | $CONH_2$ | H | Me | Me | 4-Br | 187-9 |
| 32 | $CONH_2$ | H | Me | Me | 3-Cl | 174-6 |
| 33 | $CONH_2$ | H | Me | Me | 4-Cl | 162-4 |
| 34 | $CONH_2$ | H | Me | Me | 4-$NO_2$ | 207-8 |
| 35 | $CONH_2$ | H | Me | Me | H | 164-6 |
| 36 | $CONH_2$ | H | Me | Me | 3-Me | 141-2 |
| 37 | $CONH_2$ | H | Me | Me | 4-Me | 169-71 |
| 38 | $CONH_2$ | H | Et | H | 3,4-diCl | 170-71 |
| 39 | $CONH_2$ | H | Et | H | 4-Br | 149-51 |
| 40 | $CONH_2$ | H | Et | H | 3-Cl | 168-70 |

| | $R^6$ | $R^3$ | $R^1$ | $R^2$ | X | m.p. ° |
|---|---|---|---|---|---|---|
| 41 | $CONH_2$ | H | Et | H | 4-Cl | 149-50 |
| 42 | $CONH_2$ | H | Et | H | $4-NO_2$ | 165-7 |
| 43 | $CONH_2$ | H | Et | H | H | 134-7 |
| 44 | $CONH_2$ | H | Et | H | 3-Me | 145-7 |
| 45 | $CONH_2$ | H | Et | H | 4-Me | 142-4 |
| 46 | $CO_2Me$ | H | H | H | 4-Br | 216-7 |
| 47 | $CO_2Me$ | H | Me | H | 3,4-diCl | 174-5 |
| 48 | $CO_2Me$ | H | Me | H | 4-Br | 149-50 |
| 49 | $CO_2Me$ | H | Me | H | 3-Cl | 125-6 |
| 50 | $CO_2Me$ | H | Me | H | 4-Cl | 150-52 |
| 51 | $CO_2Me$ | H | Me | H | $3-NO_2$ | 178-80 |
| 52 | $CO_2Me$ | H | Me | H | H | 127-9 |
| 53 | $CO_2Me$ | H | Me | H | $3-CF_3$ | 139-41 |
| 54 | $CO_2Me$ | H | Me | H | 3-Me | 119-21 |
| 55 | $CO_2Me$ | H | Me | H | 4-Me | 158 |
| 56 | $CO_2Me$ | H | Me | Me | 3,4-diCl | 204-6 |
| 57 | $CO_2Me$ | H | Me | Me | 4-Br | 138-40 |
| 58 | $CO_2Me$ | H | Me | Me | 3-Cl | 84-6 |
| 59 | $CO_2Me$ | H | Me | Me | $3-NO_2$ | 200-202 |
| 60 | $CO_2Me$ | H | Me | Me | H | 100-103 |
| 61 | $CO_2Me$ | H | Me | Me | $3-CF_3$ | 91-3 |
| 62 | $CO_2Me$ | H | Et | H | 3,4-diCl | 119-21 |
| 63 | $CO_2Me$ | H | Et | H | 4-Br | 144-5 |

| | $R^6$ | $R^3$ | $R^1$ | $R^2$ | X | m.p. ° |
|---|---|---|---|---|---|---|
| 64 | $CO_2Me$ | H | Et | H | 3-Cl | 109-11 |
| 65 | $CO_2Me$ | H | Et | H | 4-Cl | 146-7 |
| 66 | $CO_2Me$ | H | Et | H | H | 144-5 |
| 67 | $CO_2Me$ | H | Et | H | 3-Me | 117-20 |
| 68 | $CO_2Me$ | H | Et | H | 4-Me | 156-8 |
| 69 | $CO_2Me$ | Me | H | H | 3,4-diCl | 216-7 |
| 70 | $CO_2Me$ | Me | H | H | H | 165-6 |
| 71 | $CO_2Me$ | Me | Me | H | H | 90-92 |
| 72 | $CO_2Me$ | Me | $PhCH_2$ | H | H | 98-100 |
| 73 | $CO_2Et$ | H | H | H | 3,5-diCl | 206-7 |
| 74 | $CO_2Et$ | H | H | H | $3-NO_2$ | 160 |
| 75 | $CO_2Et$ | H | Me | H | 3,4-diCl | 143-6 |
| 76 | $CO_2Et$ | H | Me | H | 4-Br | 127-8 |
| 77 | $CO_2Et$ | H | Me | H | 3-Cl | 151-3 |
| 78 | $CO_2Et$ | H | Me | H | 4-Cl | 134-6 |
| 79 | $CO_2Et$ | H | Me | H | $3-NO_2$ | 197-9 |
| 80 | $CO_2Et$ | H | Me | H | H | 137-8 |
| 81 | $CO_2Et$ | H | Me | H | $3-CF_3$ | 154-6 |
| 82 | $CO_2Et$ | H | Me | H | 3-Me | 113-4 |
| 83 | $CO_2Et$ | H | Me | H | 4-Me | 108-9 |
| 84 | $CO_2Et$ | H | Me | H | 3-OMe | 96-7 |
| 85 | $CO_2Et$ | H | Me | H | 4-OMe | 86-8 |
| 86 | $CO_2Et$ | H | Me | H | $4-NMe_2$ | 125-8 |

BAD ORIGINAL

| | $R^6$ | $R^3$ | $R^1$ | $R^2$ | X | m.p. ° |
|---|---|---|---|---|---|---|
| 87 | $CO_2Et$ | H | Me | Me | 3,4-diCl | 145-7 |
| 88 | $CO_2Et$ | H | Me | Me | 4-Br | 119-22 |
| 89 | $CO_2Et$ | H | Me | Me | 3-Cl | 113-5 |
| 90 | $CO_2Et$ | H | Me | Me | 4-Cl | 112-4 |
| 91 | $CO_2Et$ | H | Me | Me | H | 115-30 |
| 92 | $CO_2Et$ | H | Me | Me | 3-$CF_3$ | 108-11 |
| 93 | $CO_2Et$ | H | Me | Me | 3-Me | 97-100 |
| 94 | $CO_2Et$ | H | Me | Me | 4-Me | 116-8 |
| 95 | $CO_2Et$ | H | Me | Me | 3-OMe | 130-32 |
| 96 | $CO_2Et$ | H | Me | Me | 4-OMe | 103-6 |
| 97 | $CO_2Et$ | H | Et | H | 3,4-diCl | 122-4 |
| 98 | $CO_2Et$ | H | Et | H | 4-Br | 104-12 |
| 99 | $CO_2Et$ | H | Et | H | 3-Cl | 101-2 |
| 100 | $CO_2Et$ | H | Et | H | 4-Cl | 123-4 |
| 101 | $CO_2Et$ | H | Et | H | H | 103-4 |
| 102 | $CO_2Et$ | H | Et | H | 3-Me | 124-5 |
| 103 | $CO_2Et$ | H | Et | H | 4-Me | 113-4 |
| 104 | $CO_2Et$ | H | Et | H | 3-OMe | 84-6 |
| 105 | $CO_2Et$ | H | Et | H | 4-OMe | 102-3 |
| 106 | $CO_2Et$ | H | Et | H | 4-$NMe_2$ | 112-4 |
| 107 | $CO_2Et$ | H | $PhCH_2$ | H | 4-Me | 132-5 |
| 108 | $CO_2Et$ | H | $PhCH_2$ | H | 4-OMe | 118-20 |
| 109 | $CO_2Et$ | Me | H | H | 3,5-diCl | 193-6 |

| | $R^6$ | $R^3$ | $R^1$ | $R^2$ | X | m.p. ° |
|---|---|---|---|---|---|---|
| 110 | $CO_2Et$ | Me | H | H | 4-Br | 214-5 |
| 111 | $CO_2Et$ | Me | H | H | 3-Cl | 142-4 |
| 112 | $CO_2Et$ | Me | H | H | 4-Cl | 206-7 |
| 113 | $CO_2Et$ | Me | H | H | H | 146-8 |
| 114 | $CO_2Et$ | Me | H | H | 3-$CF_3$ | 121-3 |
| 115 | $CO_2Et$ | Me | H | H | 3-Me | 136-7 |
| 116 | $CO_2Et$ | Me | H | H | 4-Me | 155-6 |
| 117 | $CO_2Et$ | Me | H | H | 3-OMe | 166-75 |
| 118 | $CO_2Et$ | Me | H | H | 4-OMe | 130 decomp |
| 119 | $CO_2Et$ | Me | H | H | 4-$NMe_2$ | 165-70 |
| 120 | $CO_2Et$ | Me | Me | H | 4-Br | 158-9 |
| 121 | $CO_2Et$ | Me | Me | H | 4-Cl | 127-9 |
| 122 | $CO_2Et$ | Me | Me | H | 3-$NO_2$ | 112-3 |
| 123 | $CO_2Et$ | Me | Me | H | H | 80-81 |
| 124 | $CO_2Et$ | Me | Me | H | 3-$CF_3$ | 108-9 |
| 125 | $CO_2Et$ | Me | Me | H | 3-Me | oil |
| 126 | $CO_2Et$ | Me | Me | H | 3-OMe | 112-4 |
| 127 | $CO_2Et$ | Me | Me | H | 4-OMe | 90-92 |
| 128 | $CO_2Et$ | Me | Me | H | 4-$NMe_2$ | 132-3 |
| 129 | $CO_2Et$ | Me | Me | Me | 4-Br | 150-51 |
| 130 | $CO_2Et$ | Me | Me | Me | 3-$NO_2$ | 153-4 |
| 131 | $CO_2Et$ | Me | Me | Me | 3-$CF_3$ | 115-6 |
| 132 | $CO_2Et$ | Me | $PhCH_2$ | H | 3,5-diCl | 103 |

| | $R^6$ | $R^3$ | $R^1$ | $R^2$ | X | m.p. ° |
|---|---|---|---|---|---|---|
| 133 | $CO_2Et$ | Me | $PhCH_2$ | H | 4-OMe | 111-2 |
| 134 | $CO_2Et$ | Et | H | H | H | 128-9 |
| 135 | $CO_2Et$ | Et | Me | H | H | 83-4 |
| 136 | $CO_2CHMe_2$ | Me | Me | H | H | 90-92 |

Example 25 (Intermediates)

Ethyl 3-[methyl-(3,4-dichlorophenyl)-amino]-2-cyano-3-(methylthio)-propenoate

Ethyl 3-(3,4-dichlorophenylamino)-2-cyano-3-(methylthio)-propenoate (11g), dimethyl sulphate (4.2g), potassium carbonate, anhyd (9.2g), and acetone (50 ml) were stirred together and heated under reflux for 20 hours. The mixture was filtered and the filtrate was evaporated to leave an oil which crystallised on trituration with 40-60° petrol. Recrystallisation from ethanol gave the title compound (7.2g, 63%) m.p. 106-8°.

The compounds shown in the following Table VIII were also made by this process:

Table VIII

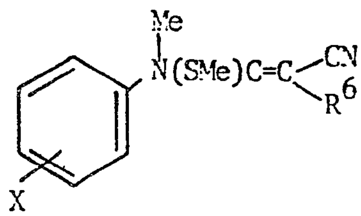

| | $R^6$ | X | m.p. ° |
|---|---|---|---|
| 1 | CN | 4-Br | 130-31 |
| 2 | CN | H | 95-6 |
| 3 | $CO_2Me$ | 3,4-diCl | 106-8 |
| 4 | $CO_2Me$ | H | 84-5 |
| 5 | $CO_2Et$ | 3,5-diCl | 126-7 |
| 6 | $CO_2Et$ | 4-Br | 108-10 |
| 7 | $CO_2Et$ | 3-Cl | 68-70 |
| 8 | $CO_2Et$ | 4-Cl | 114-5 |
| 9 | $CO_2Et$ | 4-F | 103-5 |
| 10 | $CO_2Et$ | $3-NO_2$ | 96-8 |
| 11 | $CO_2Et$ | H | 78-9 |
| 12 | $CO_2Et$ | $3-CF_3$ | 79-81 |
| 13 | $CO_2Et$ | 3-Me | 75-7 |
| 14 | $CO_2Et$ | 4-Me | 122-3 |
| 15 | $CO_2Et$ | 3-OMe | 99-100 |
| 16 | $CO_2Et$ | 4-OMe | 96-8 |
| 17 | $CO_2Et$ | $4-NMe_2$ | 121-4 |
| 18 | $CO_2CHMe_2$ | H | 92-3 |

BAD ORIGINAL

Example 26 (Intermediates)

Ethyl 3-[ethyl-(phenyl)-amino]-2-cyano-3-(methylthio)-propenoate

Ethyl 3-phenylamino-2-cyano-3-(methylthio)-propenoate (19.6g), iodethane (13.4g), potassium carbonate, anhyd (20.7g), and acetone (100 ml) were stirred and heated under reflux for 24 hours. The mixture was filtered and the filtrate was evaporated. Recrystallisation of the residue gave the title compound (12.6g), m.p. 70-72°.

Example 27 (Intermediate)

4-Chloro-3-methylaniline

(a) 1-Chloro-2-methyl-4-nitrobenzene

Sodium nitrite (69g, 1M) in water (530 ml) was added slowly to 2-methyl-4-nitroaniline (152g, 1M) in a mixture of concentrated hydrochloric acid (270 ml) and water (600 ml) at < 10°C. The solution was stirred for 10 minutes and then added to a solution of cuprous chloride (129g) in concentrated hydrochloric acid (510 ml) at < 20°C. The mixture was stirred at room temperature for 1 hour and then the product was filtered off and recrystallised from ethanol. (Yield 149g, 87%), m.p. 40-44°C.

(b) 4-Chloro-3-methylaniline

The product of step (a) (149g), iron filings (365g), 2-propanol (2½ l), water (250 ml) and concentrated hydrochloric acid (70 ml) were stirred and heated under reflux for 3 hours. The mixture was filtered and the filtrate was evaporated. The reaction product was washed with dilute aqueous sodium hydroxide,

BAD ORIGINAL

then water, and recrystallised from ethanol to give the desired product (52.3g, 43%), m.p. 81-4°.

Example 28

Potassium salt of ethyl dicyanoacetate

A solution of malononitrile (330g, 5M) and ethyl chloroformate (425 ml, 5.25M) in tetrahydrofuran (500 ml) was added slowly to a solution of potassium hydroxide (560g, 10M) in water (2l) at 30-40°. The mixture was stirred at room temperature for an hour and then cooled to 0°C. The precipitated product was filtered off and washed with water and with ethanol. (415.5g, 47%), m.p. 297-8°.

| | | | | |
|---|---|---|---|---|
| | Found: | C, 40.75; | H, 3.15; | N, 15.7 |
| $C_6H_5N_2O_2K$ | requires: | C, 40.9; | H, 2.85; | N, 15.9% |

Example 29

Ethyl 3-amino-3-[methyl-(3-nitrophenyl)-amino]-2-cyanopropenoate

(a) m-Nitroaniline (13.8g), the product of Example 28 (19g), concentrated hydrochloric acid (10 ml) and water (70 ml) were heated under reflux for 48 hours. The solid was filtered off, washed well with ethanol and recrystallised from 2-methoxyethanol. (7.5g, 26%), m.p. 160°.

| | | | | |
|---|---|---|---|---|
| | Found: | C, 51.8; | H, 4.1; | N, 20.3 |
| $C_{12}H_{12}N_4O_4$ | requires: | C, 52.15; | H, 4.4; | N, 20.3% |

(b) The product of step (a) (5g), iodomethane (2.6g), potassium carbonate anhydrous (1.5g) and acetone (35 ml) were heated under reflux for 12 hours. The mixture was filtered and the filtrate

was evaporated. The mixture was recrystallised from ethanol to give the desired product (2g, 38%), m.p. 166-8°.

| | | | |
|---|---|---|---|
| Found: | C, 53.55; | H, 5.05; | N, 19.6 |
| $C_{13}H_{14}N_4O_4$ requires: | C, 53.8; | H, 4.85; | N, 19.3% |

This compound was also prepared using the method of Example 24.

Example 30

1,3-Bis-methylamino-1-chlorophenylamino-2-cyano-3-imino-propene

3-Chlorophenylamino-(methylthio)-methylenepropanedinitrile (8.3g), methylamine (3.5g), and ethanol (30 ml) were heated under reflux for 24 hours. The mixture was cooled to precipitate the title product (3g, 34%), m.p. 189-91°.

| | | | |
|---|---|---|---|
| Found: | C, 54.85; | H, 5.25; | N, 26.25 |
| $C_{12}H_{14}ClN_5$ requires: | C, 54.65; | H, 5.35; | N, 26.55% |

Example 31

(a) Ethyl 3-t-butylamino-2-cyano-3-methylthiopropenoate

Ethyl 2-cyano-3,3-dimethylthiopropenoate (108.5g), t-butylamine (40g) and ethanol (200 ml) were heated under reflux for 4 hours and then cooled to precipitate the sub title product (70g, 58%), mp 95-6°.

(b) Ethyl 3-t-butylamino-2-cyano-3-methylaminopropenoate

The product from (a) (8.1g), 27% aqueous methylamine (10 ml), and ethanol (30 ml) were heated under reflux for 8 hours. The mixture was cooled and the precipitate was recrystallised from ethanol to



BAD ORIGINAL

give the title product (6.8g), mp 149-50°.

Example 32

(a) Ethyl 2-cyano-3-(2-methylpropylamino)-3-methylthiopropenoate m.p. 15-17° was prepared by the method of Example 31 (a).

(b) Ethyl 2-cyano-3-(N-methyl-2-methylpropylamino)-3-methylthiopropenoate

The product from step (a) (24.2g), potassium carbonate (27.6g), dimethylsulphate (12.5g), and acetone (120 ml) were stirred and heated under reflux for 20 hours. The mixture was filtered. The filtrate was evaporated and the residue was recrystallised from ethanol to give the title product (12g, 47%), mp 52-5°.

(c) Ethyl 3-amino-2-cyano-3-(N-methyl-2-methylpropylamino)-propenoate

The product from step (b) (8.5g), ethanol (30 ml) and .880 ammonia (20 ml) were heated under reflux for 24 hours. Ammonia gas was passed through the reaction mixture for the last 8 hours of this time. The ethanol was evaporated off and the residue was recrystallised from ethyl acetate/80-100° petroleum ether to give the title product (3.7g), m.p. 58-9°.

| | | | | |
|---|---|---|---|---|
| | Found: | C, 58.4; | H, 8.25; | N, 18.45% |
| $C_{11}H_{19}N_3O_2$ | requires: | C, 58.65; | H, 8.5; | N, 18.65% |

Example 33

t-Butyl 3-amino-2-cyano-3-methyl(phenyl)amino propenoate m.p. 186-7° was prepared by the method of Example 32(c) using the appropriate starting material.



BAD ORIGINAL

Example 34

Decyltrimethylammonium bromide (100 parts) was dissolved in warm water (1,000 parts). The solution was cooled and chloroform (750 parts) was added to produce an emulsion-like liquid. A freshly prepared and filtered solution of potassium tricyanomethanide (52 parts) in water (150 parts) was added and the mixture was shaken and allowed to separate. The lower chloroform layer was separated and the aqueous layer re-extracted with chloroform. The chloroform extracts were combined and washed with a 5% aqueous solution of potassium tricyanomethanide, dried over magnesium sulphate and evaporated under reduced pressure to give a golden yellow oil of decyltrimethylammonium tricyanomethanide (88 parts, 85% yield).

| | | | |
|---|---|---|---|
| Found: | C, 70.15; | H, 10.25; | N, 19.55% |
| $C_{17}H_{30}N_4$ requires: | C, 70.3; | H, 10.4; | N, 19.3% |

Example 35

Concentrated hydrochloric acid was added to a suspension of 2-(methylamino)quinoxaline (100 parts) in water (625 parts) until a clear solution was obtained. A solution of potassium tricyanomethanide (82 parts) in warm water (125 parts) was added. An immediate yellow precipitate formed. The mixture was cooled to $0^o$ and the precipitate was filtered off, recrystallised from water and dried to give the 2-(methylamino)quinoxaline salt of tricyanomethane (110 parts, 70% yield) as its hydrate, mp 166-$8^o$.

Found: C, 58.0; H, 4.4; N, 31.75%

$C_{13}H_{12}N_6O$ requires: C, 58.2; H, 4.5; N, 31.4%

Example 36

The following new salts (all of tricyanomethane) were prepared by methods analogous to those described in Examples 34 and 35. All had satisfactory elemental analysis.

1. hexadecyltrimethylammonium mp 50-7°
2. 1-(2,4-dinitrophenyl)pyridinium mp 138-140°
3. benzyltrimethylammonium mp 115-7°
4. (o-(bromomethyl)benzyl)trimethylammonium mp 178-9°
5. triethyloctylammonium, liquid
6. tetrabutylammonium mp 45-8°
7. trimethyloctadecylammonium mp 64-6°
8. trimethyloctylammonium, liquid
9. dodecyltrimethylammonium mp 29-30°
10. triethylheptylammonium, liquid
11. trimethyltetradecylammonium mp 42-5°
12. 1-hexadecylpyridinium mp 49-53°
13. trimethyl (o-(thiocyanatomethyl)benzyl)ammonium mp 66-8°
14. triethyloctadecylammonium mp 52-3°
15. decyltriethylammonium, liquid
16. trimethylnonylammonium, liquid
17. heptyltrimethylammonium, liquid
18. o-phenylenedimethylenebis[trimethylammonium] mp of dihydrate 172-4°

19. dodecyltriethylammonium, liquid

20. butyltriethylammonium, liquid

21. N,N,N-triethyl-N',N',N'-trimethyl-N,N'-*o*-phenylenedimethylene-bis[ammonium], mp 85-7°

22. 4,5-dimethyl-*o*-phenylenedimethylenebis[trimethylammonium] mp 138-140°

23. 3,4,5,6-tetrahydro-7-(isopropylamino)-2-*H*-azepine salt, mp 53-5°

24. octylamine salt, mp 122-3°

25. 1,1'-dimethyl-4,4'-bipyridylium bis salt, mp 188-189°

26. decylamine salt, mp 125-7°

27. 3,4-dichloroaniline salt mp 225-230°

28. piperidine salt mp 75-6°

29. morpholine salt mp 120°

30. *m*-(trifluoromethyl)aniline salt mp 197-9°

31. 2,5-dichloroaniline salt mp 188-192°

32. 5,6-dichlorobenzimidazole salt decomposed at 230°

33. aniline salt mp 135-8°

34. hexylamine salt mp 80-2°

35. 2-amino-3-hydroxypyridine salt mp 170° (decomposed)

36. 2-amino-6-methylpyridine salt mp 99-100°

37. 2-methoxyaniline salt mp 174-6°

38. 2-iodoaniline salt mp 122° (decomposed)

39. 2-bromoaniline salt mp 194-6°

40. 3-aminopyridine salt (1:2) dihydrate, mp 130° (decomposed)

41. 3-chloroaniline salt mp 214-6°
42. 2-aminopyridine salt mp 104-5°
43. 4-chloro-2-methylaniline salt mp 160° (decomposed)
44. 2-ethylpiperidine salt mp 79-80°
45. 5-chloro-2-methylaniline salt mp 215-7°
46. 2,6-dimethylmorpholine salt mp 137-8°
47. hexahydroazepine salt mp 125-7°
48. 2-chloro-6-methylaniline salt mp 125° (decomposed)
49. 4-phenoxyaniline salt mp 205°
50. 3-chloro-2-methylaniline salt mp 150° (decomposed)
51. cyclohexylamine salt mp 200-1°
52. octadecylamine salt mp 135-6°
53. N-Butyl-N-ethylammonium salt, oil.
54. dipropylammonium salt, mp 55-60°
55. N-ethyl-N-phenylammonium salt mp 60° decomp
56. 2-bromophenylammonium salt mp 191.5°
57. 2-iodophenylammonium salt mp 117-9°
58. 4-chloro-2-methylphenylammonium salt mp 160°.

Also produced by the same method was the piperidinium salt of ethyl dicyanoacetate - oil

Example 37

Potassium salt of isopropyl dicyanoacetate

A solution of malononitrile (15.9g, 0.24M) in 2-propylchloroformate (29.5g, 0.24M) was added slowly, with stirring, to 5M aqueous potassium hydroxide (96.5 ml, 0.428M). Ice cooling was used to keep the

BAD ORIGINAL

temperature below 45°C. Addition took 20 minutes. The solution was stirred at 35-45°C for a further 20 minutes and then cooled to 0°C. The precipitate was washed with ice-cold water and with 2-propanol, and dried to give the potassium salt of isopropyl dicyanoacetate (18.1g, 40%).

Example 38

(a) Potassium salt of n-butyldicyanoacetate

A solution of malononitrile (19.8g) and n-butyl chloroformate (45g) in tetrahydrofuran (30 ml) was added slowly to 5M aqueous potassium hydroxide (120 ml) with ice-water cooling to keep the temperature below 40°C, addition took about 20 minutes. The mixture was stirred at room temperature for 1 hour and then cooled to 0°. The precipitate was filtered off and recrystallised from ethanol to give the title product (35g, 58%), mp 250° decomp.

Prepared similarly and recrystallised from water were the potassium salts of:-

(b) 2-Methylpropyl dicyanoacetate mp 298-300°

(c) S-Ethyl dicyanoethanethioate mp 285° decomp

Potassium salts of the following were prepared similarly, but isolated by evaporating the reaction mixture to dryness, extracting the residue with acetone, evaporating the acetone and recrystallising the residue from water:-

(d) n-Propyl dicyanoacetate m.p. 235-7°

(e) 2-Methylbutyl dicyanoacetate mp 270-72°

(f) 1-Methylhexyl dicyanoacetate mp 207-9°

(g) 1-Methylpropyl dicyanoacetate mp 278-80°

Example 39

(a) Potassium salt of N-methyldicyanoacetamide

A solution of propanedinitrile (6g, 0.09M) and methyl isocyanate (6 ml, 5.8g, 0.1M) in tetrahydrofuran (10 ml) was added, with stirring, to one of potassium hydroxide (5.1g, 0.09M) in water (40 ml) over a period of 10 minutes at 25-30° (water cooling). The pH of the red solution, after addition, was 8. After 1 hour, the solution was evaporated to dryness and the residue was recrystallised from 50:50 2-propanol:water to give the title compound (2g, 14%), mp 294-5° decomp.

(b) Potassium salt of N-ethyl-dicyanoacetamide

mp 270° (decomp) was prepared similarly.

Example 40

Triethylamine salt of N-phenyl dicyanoacetamide

Triethylamine (70 ml, 51g, 0.505M) was added slowly to a solution of propanedinitrile (33g, 0.5M) and phenylisocyanate (59.5g, 0.5M) in tetrahydrofuran (200 ml), with ice cooling to keep the temperature below 40°. After addition, the mixture was cooled to 3° to precipitate the product, mp 93-4°.

Example 41

Pyridine salt of N-methyldicyanoacetamide

Methyl isocyanate (6 ml, 5.8g, 0.1M) was added to a solution of propanedinitrile (6g, 0.09M) in pyridine (18 ml). Over a period of



BAD ORIGINAL

15 minutes the solution became warm and turned dark red. It was left at room temperature overnight. The dark red, crystalline precipitate was filtered off, washed with ethanol and recrystallised from ethanol to give pale orange crystals of the title compound (12.3g, 67%), mp 141-2$^{o}$.

Example 42

Triethylamine salt of N-methyldicyanoacetamide

A mixture of propanedinitrile (6g, 0.09M), triethylamine (15 ml, 11g, 0.11M), and toluene (25 ml) was warmed until the propanedinitrile melted. Methyl isocyanate (6 ml, 5.8g, 0.1M) was added, with water cooling, and the mixture was left at room temperature overnight. The precipitated title compound was filtered off, toluene-washed and recrystallised from ethanol. (Yield 10g, 45%) mp 139-41$^{o}$.

Example 43

Dodecylguanidine salt of tricyanomethane

Potassium salt of tricyanomethane (15g) in hot water (25 ml) was added to dodecylguanidinium acetate (28.6g) in hot water (140 ml). The mixture was cooled. The oil which had separated crystallised rapidly. The solid was filtered off and washed with water to give the title product (30g, 94%), mp 46-50$^{o}$C.

| | | | |
|---|---|---|---|
| Found: | C, 64.3; | H, 9.7; | N, 26.0% |
| $C_{17}H_{30}N_6$ requires: | C, 64.1; | H, 9.5; | N, 26.4% |

Example A

The compound under test formulated as an attaclay/sand dust was incorporated in John Innes I potting compost at a rate

equivalent to 130 and 26 parts per million weight/volume of active ingredient to soil and placed in anodised aluminium pans, 19 cm long x 9.5 cm wide x 5.0 cm deep. These rates are approximately equivalent to a soil surface application of 56 and 11.2 kg active ingredient/hectare cultivated to a depth of 5 cm. Seeds of Peas, Mustard, Linseed, Maize, Oats and Ryegrass were sown in the treated soil, watered and placed in a controlled environment room ($22^{o}C$; 65-85% RH ; 14 hours artificial illumination at 1200 foot candles) for 21 days. The plants were then visually assessed for any growth regulatory or herbicidal effects. All differences from an untreated control were scored on a scale from 0 - 100, where 0 signifies no effect and 100 signifies complete suppression. The results are summarised in the following table:

BAD ORIGINAL

| Compound of Ex No | Dose in kg/ha | Peas | Mustard | Linseed | Ryegrass | Oats | Maize |
|---|---|---|---|---|---|---|---|
| 18(c) | 11.2 | 100 | 100 | 100 | 100 | 100 | 100 |
| 18(15) | 11.2 | 90 | 100 | 100 | 100 | 100 | 80 |
| 18(17) | 11.2 | 5 | 100 | 100 | 90 | 40 | 25 |
| 22(12) | 11.2 | 70 | 90 | 100 | 90 | 0 | 70 |
| 18(18) | 11.2 | 80 | 100 | 100 | 100 | 100 | 90 |
| 18(20) | 11.2 | 35 | 100 | 40 | 70 | 0 | 0 |
| 18(16) | 11.2 | 10 | 100 | 90 | 70 | 35 | 30 |
| 24(114) | 11.2 | 40 | 100 | 30 | 100 | 80 | 50 |
| | 56 | 90 | 100 | 100 | 100 | 70 | 70 |
| 24(115) | 11.2 | 100 | 100 | 100 | 100 | 100 | 70 |
| | 56 | 100 | 100 | 100 | 100 | 100 | 100 |
| 24(113) | 11.2 | 100 | 100 | 100 | 100 | 100 | 70 |
| | 56 | 100 | 100 | 100 | 100 | 100 | 85 |

Example B

The compound under test was formulated as: (I) an attaclay/sand dust and incorporated in John Innes I potting compost at a rate equivalent to 6.5 parts per million weight/volume of active ingredient to soil and placed in anodised aluminium pans, 19 cm long x 9.5 cm wide x 5.0 cm high. This is approximately equivalent to a surface application of 2.8 kg active ingredient per hectare cultivated to a depth of 5 cm. Seeds of the species listed below were sown in the treated soil, one species per pan. (II) An aqueous

suspension together with 1000 ppm of the wetting agent Lissapol NX. The surfaces of an additional set of pans with seeds already sown were then sprayed with 2.8 kg/ha in 450 litres/hectare.

All the pans were watered and placed in a controlled environment room (22$^{o}$C ; 65-85% RH and 14 hours artificial illumination at 1600 foot candles) for 21 days. The plants were then visually assessed for any growth regulatory or herbicidal effects. All differences from an untreated control were scored on a scale from 0 - 100, where 0 signifies no effect and 100 signifies complete suppression. The results are summarised in the following table:

| Species | | Soil Incorporation Compound of Ex 18c | 18 (15) | 18 (17) | 18 (18) | Ex 24 (115) | Ex 24 (113) | Surface Spray Compound of Ex 18 (c) | 18 (15) | 18 (17) | 18 (18) | Ex 24 (115) | Ex 24 (113) |
|---|---|---|---|---|---|---|---|---|---|---|---|---|---|
| Chickweed | - (Stellaria media) | 100 | 100 | 90 | 100 | 100 | 100 | 100 | 80 | 70 | 100 | 100 | 100 |
| Mustard | - (Sinapis alba) | 100 | 100 | 60 | 100 | 100 | 100 | 100 | 90 | 30 | 90 | 30 | 100 |
| Cotton | - (Gossypium sp) | 100 | 20 | 0 | 90 | 0 | 20 | 20 | 0 | 20 | 80 | 0 | 0 |
| Tomato | - (Lycopersicon esculentum) | 100 | 100 | 100 | 100 | 100 | 100 | 100 | 80 | 90 | 100 | 100 | 100 |
| Fathen | - (Chenopodium album) | 100 | 100 | 90 | 100 | 100 | 100 | 100 | 90 | 70 | 100 | 100 | 100 |
| Carrot | - (Daucus carota) | 100 | 40 | 0 | 40 | 80 | 100 | 100 | 30 | 20 | 70 | 20 | 90 |
| Wheat | - (Triticum aestivum) | 100 | 100 | 70 | 100 | 100 | 100 | 100 | 70 | 0 | 90 | 100 | 100 |
| Barley | - (Hordeum vulgare) | 100 | 100 | 30 | 100 | 100 | 100 | 100 | 90 | 0 | 90 | 100 | 100 |
| Wild Oat | - (Avena fatua) | 100 | 100 | 30 | 100 | 100 | 100 | 100 | 90 | 0 | 100 | 100 | 100 |
| Blackgrass | - (Alopecurus myosuroides) | 100 | 100 | 80 | 100 | 100 | 100 | 100 | 100 | 60 | 100 | 100 | 100 |
| Barnyardgrass | - (Echinochloa crus-galli) | 100 | 100 | 70 | 100 | 100 | 100 | 100 | 100 | 5 | 90 | 70 | 100 |
| Crabgrass | - (Digitaria sanguinalis) | 100 | 100 | 70 | 100 | 100 | 100 | 100 | 100 | 70 | 100 | 80 | 100 |

Example C

Seeds of Peas, Mustard, Linseed, Ryegrass, Sugarbeet, Oats and French beans were sown in anodised aluminium pans, 19 cm long x 9.5 cm wide x 5 cm deep containing John Innes I potting compost. They were then watered and placed in a controlled environment room (22°C ; 65-85% RH ; 14 hours artificial illumination at 1200 foot candles). Fourteen days after sowing the seedlings received a foliar spray of the compound under test formulated as an aqueous suspension together with 1000 ppm of the wetting agent Lissapol NX. The concentrations of active ingredient and volume of application were adjusted so as to be equivalent to rates of 11.2 and 2.8 kg/ha in 450 litres per hectare. After seven days growth in a controlled environment room the plants were visually assessed for any herbicidal or growth regulant response. All differences from the untreated control were scored according to a herbicidal index where 0 = no effect and 100 = complete kill. The results are summarised in the following table:

| Compound of Ex No | Dose in kg/ha | Peas | Mustard | Linseed | Ryegrass | Oats | Beet | F.Beans |
|---|---|---|---|---|---|---|---|---|
| 18(c) | 2.8 | 5 | 100 | 25 | 100 | 90 | 100 | 100 |
| 18(15) | 2.8 | 5 | 100 | 35 | 80 | 80 | 100 | 100 |
| 18(17) | 2.8 | 0 | 80 | 10 | 0 | 0 | 70 | 0 |
| 22(12) | 2.8 | 15 | 80 | 35 | 35 | 20 | 90 | 10 |
| 18(19) | 2.8 | 0 | 15 | 35 | 0 | 0 | 70 | 10 |
| 18(18) | 2.8 | 10 | 100 | 70 | 60 | 5 | 90 | 80 |
| 18(21) | 2.8 | 5 | 90 | 60 | 0 | 0 | 80 | 5 |
| 18(20) | 2.8 | 5 | 100 | 25 | 0 | 0 | 100 | 5 |
| 18(14) | 2.8 | 5 | 40 | 5 | 30 | 0 | 90 | 40 |
| 18(16) | 2.8 | 5 | 60 | 15 | 0 | 0 | 70 | 5 |
| 24(115) | 2.8 | 10 | 100 | 20 | 40 | 10 | 100 | 50 |
| | 11.2 | 100 | 100 | 100 | 100 | 100 | 100 | 100 |
| 24(113) | 2.8 | 40 | 100 | 85 | 90 | 100 | 100 | 100 |
| | 11.2 | 100 | 100 | 100 | 100 | 100 | 100 | 100 |

Example D

Seeds of various dicotyledon species, listed below were sown in anodised aluminium pans, 19 cm long x 9.5 cm wide x 5.0 cm deep and placed in a controlled environment room (22$^{o}$C; 65-85% RH; 14 hours artificial illumination at 1600 foot candles). When all species had cotyledons plus at least two fully expanded true leaves they received a foliar spray of the compounds under test formulated as aqueous

BAD ORIGINAL

suspension together with 1000 ppm of the wetting agent Lissapol NX. One pan of each species received the equivalent of 1.4 or 0.7 kg in 450 litres/hectare and were returned to the controlled environment room.

Fourteen days after treatment the plants were visually assessed for any growth regulatory or herbicidal effect. All differences from an untreated control were scored on a scale from 0-100 where 0 signifies no effect and 100 signifies complete suppression.

The results are summarised in the following table:

| Species | | Compound of Ex 18(c) | | Ex 18(15) | | Ex 18(18) | |
|---|---|---|---|---|---|---|---|
| | Dosage rate kg/ha | 1.4 | 0.7 | 1.4 | 0.7 | 1.4 | 0.7 |
| Chickweed | - (Stellaria media) | 100 | 100 | - | 30 | 90 | 90 |
| Mayweed | - (Matricaria spp) | 25 | 20 | - | 20 | 10 | 10 |
| Cleavers | - (Galium aparine) | 100 | 100 | - | 25 | 90 | 70 |
| Fathen | - (Chenopodium album) | 100 | 100 | - | 80 | 100 | 100 |
| Corn marigold | - (Chrysanthemum segetum) | 0 | 20 | - | 25 | 40 | 30 |
| Pale persicaria | - (Polygonum lapathifolium) | 100 | 90 | - | 25 | 90 | 80 |
| Pigweed | - (Amaranthus retroflexus) | 100 | 80 | - | 5 | 100 | 100 |
| Wheat | - (Triticum aestivum) | 80 | - | 80 | - | 40 | - |
| Barley | - (Hordeum vulgare) | 90 | - | 90 | - | 80 | - |
| Wild Oat | - (Avena fatua) | 90 | - | 40 | - | 30 | - |
| Blackgrass | - (Alopecurus myosuroides) | 90 | - | 70 | - | 45 | - |
| Barnyardgrass | - (Echinochloa crus-galli) | 100 | - | 50 | - | 60 | - |
| Crabgrass | - (Digitaria sanguinalis) | 100 | - | 70 | - | 30 | - |

BAD ORIGINAL

Example E

Aqueous solutions or suspensions containing 2,000, 500 or 125 ppm of the compounds listed below and 125 ppm of nonylphenol/ethylene oxide condensate as wetting agent were sprayed until the foliage was completely wetted on to young French bean plants (*phaseolus vulgaris*) having two fully expanded leaves. After 24 hours, the treated plants were inoculated with an aqueous suspension of spore of the disease organism bean rust (*Uromyces phaseoli*). The plants were then placed in a water saturated atmosphere for 24 hours and then kept in a controlled environment room (temperature 18$^{o}$, relative humidity 80-90%) for 14 days, after which the incidence of fungal disease was measured. The percentage disease control in comparison with plants sprayed with a solution of the wetting agent alone is tabulated below:

| Compound of Example No | 500 ppm | 125 ppm |
|---|---|---|
| 36(i) | 100 | 40 |
| 43 | 94 | 61 |

Example F

Aqueous solutions or suspensions containing 2000, 500 or 125 ppm of the compounds listed below and 125 ppm of nonylphenol/ethylene oxide condensate as wetting agent were sprayed until the foliage was completely wetted on to young bean plants (*Vicia fabae*) having two fully expanded leaves. After 24 hours, the treated plants were inoculated with an aqueous suspension of spores of the disease organism chocolate spot (*Botrytis fabae*). The plants were then



BAD ORIGINAL

placed in a water saturated atmosphere for five days after which the incidence of fungal disease was measured. The percentage disease control in comparison with plants sprayed with a solution of the wetting agent alone is tabulated below:

| Compound of Example No | 500 ppm | 125 ppm |
|---|---|---|
| 36(1) | 91 | 80 |
| 36(28) | 84 | |
| 36(29) | 55 | |
| 43 | 91 | 66 |

Example G

10 ml of an aqueous acetone suspension of each compound listed in the table below at a concentration of 2000, 500 or 125 parts per million (ppm) as listed in the table were applied around the base of French bean plants, Phaseolus vulgaris, whose first cotyledons had fully expanded. 1 Day later, the plants were inoculated with spores of bean rust (Uromyces phaseoli), placed in a water saturated atmosphere for 24 hours, and then transferred to a controlled environment ($18^{o}$, 80-90% relative humidity) for 14 days. The incidence of fungal disease was then measured. The percentage disease control is tabulated below:

| Compound of Example No | 2000 ppm | 500 ppm | 125 ppm |
|---|---|---|---|
| 36(28) | 95 | 90 | 90 |
| 36(29) | 100 | 100 | 90 |
| 36(1) | | 55 | |
| 43 | | 88 | 21 |

Example H (formulations)

(a) Wettable powder

| | |
|---|---|
| Compound of formula I | 25% or 50% w/w |
| Sodium lignosulphonate | 5% w/w |
| China clay | to 100% w/w |

Mix components and then micronise.

(b) Wettable powder

| | |
|---|---|
| Compound of formula I | 50% w/w |
| Sodium oleoyl N-methyl tauride | 5% w/w |
| A sulphonated condensate (Dyapol PFS) | 3% w/w |
| China clay | 42% w/w |

Mix components and then micronise.

(c) Emulsifyable concentrate

| | |
|---|---|
| Compound of formula I | 10% w/v |
| Castor oil ethoxylate 40 moles | 5% w/v |
| Isophorone | to 100% w/v |

Dissolve components in isophorone and filter.

(d) Seed dressing

| | |
|---|---|
| Compound of formula I | 98% w/w |
| High boiling aliphatic mineral oil | 2% w/w |

(e) Seed dressing

| | |
|---|---|
| Compound of formula I (ground) | 50% w/w |
| China clay | 47% w/w |
| High boiling aliphatic mineral oil | 3% w/w |

The compound of formula I is mixed with the china clay and the oil added dropwise and the whole roller mixed.

(f) Wettable powder

| | |
|---|---|
| Compound of formula I | 20% w/w |
| Ethoxylated fatty alcohol absorbed on fine silica | 10% w/w |
| Polyvinyl alcohol | 5% w/w |
| China clay | 65% w/w |

Components mixed, milled and micronised.



BAD ORIGINAL

What we claim is:-

1. A fungicidal or herbicidal composition comprising a compound of formula I,

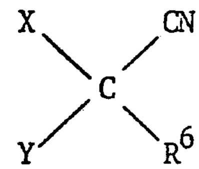

I

in which X represents a group -CN and Y a cation, or X and Y together form a group of formula II,

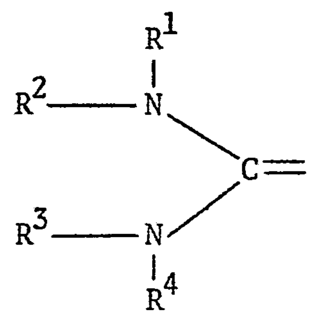

II

in which $R^1$ and $R^2$, which may be the same or different, each represents hydrogen, alkyl, cycloalkyl or benzyl,

$R^3$ and $R^4$, which may be the same or different, each represent hydrogen, alkyl, cycloalkyl, phenyl or phenyl substituted by one or more of halogen, alkyl, alkoxy, phenoxy, nitro, amino, $-COOR^9$, $-SO_3R^9$ or $-CF_3$, or $R^3$ and $R^4$ together form a 4, 5 or 6 membered methylene chain which may be fused to a benzene ring, or which may be interrupted by an oxygen atom or by a group -NH-,

$R^6$ is -CN, $-C(=NH)-NHR^8$, $-CONR^9R^{10}$, or $-CQZR^9$,

$R^8$ represents alkyl or cycloalkyl,

$R^9$ and $R^{10}$, which may be the same or different, each represent hydrogen, alkyl, phenyl or cycloalkyl, and

Q and Z, which may be the same or different, each represent oxygen or sulphur,

or a suitable derivative thereof,

provided that when $R^6$ is $-COOC_2H_5$ and $R^1$, $R^2$ and $R^3$ are all hydrogen, then $R^4$ is other than alkyl containing 3, 4 or 5 carbon atoms.

as active ingredient, together with an agriculturally acceptable adjuvant, diluent or carrier.

2. A composition according to Claim 1 comprising a surface active agent and from 0.5-85% of active ingredient.

3. A compound of formula I, as defined in Claim 1, or a suitable derivative thereof, with the provisos that

A. when X and Y together form a group of formula II,

and (b) when $R^6$ is -CN

(i) then not all of $R^1$, $R^2$, $R^3$ and $R^4$ are hydrogen,

(ii) then not all of $R^1$, $R^2$, $R^3$ and $R^4$ are methyl,

(iii) ~~and~~ when $R^1$ and $R^3$ are both hydrogen, then both of $R^2$ and $R^4$ are not octadecyl, or both of $R^2$ and $R^4$ are not p-nitrophenyl,

(iv) and when $R^1$, $R^2$ and $R^3$ are all hydrogen, then $R^4$ is not methyl or cyclohexyl,

(v) and when $R^1$ and $R^2$ are both methyl, then $R^3$ and $R^4$ are not both ethyl or propyl or do not together form a $-(CH_2)_2-O-(CH_2)_2-$ chain, and, when $R^3$ is hydrogen, $R^4$ is not butyl, phenyl or



BAD ORIGINAL

p-chlorophenyl,

(vi) and when $R^1$ and $R^2$ are both hydrogen, then $R^3$ and $R^4$ are not both ethyl or propyl or do not together form a $-(CH_2)_4-$, or a $-(CH_2)_5-$ chain, and, when $R^3$ is hydrogen or methyl, then $R^4$ is not hydrogen, ethyl, butyl, phenyl, o-methylphenyl or m-nitrophenyl,

(vii) and when $R^3$ and $R^4$ are both hydrogen and $R^1$ is hydrogen or ethyl, then $R^2$ is not benzyl, and

(c) when $R^6$ is $-COOCH_3$

(i) not all of $R^1$, $R^2$, $R^3$ and $R^4$ are methyl,

(ii) and when $R^1$, $R^2$ and $R^3$ are all hydrogen, then $R^4$ is not alkyl C 1 to 6, cyclohexyl or phenyl,

(iii) and when $R^1$, $R^3$, and $R^4$ are all hydrogen, then $R^2$ is not benzyl, and

(d) when $R^6$ is $-COOC_2H_5$

(i) and when $R^1$, $R^2$ and $R^3$ are all hydrogen, then $R^4$ is not methyl or alkyl C 3 to 6 inclusive,

(ii) and when $R^1$ and $R^2$ are both hydrogen, then $R^3$ and $R^4$ do not together form a $-(CH_2)_4-$ chain, and

(e) when $R^6$ is $-CONH_2$, then not all of $R^1$, $R^2$, $R^3$ and $R^4$ are hydrogen, and

(f) when $R^6$ is $-COOCH(CH_3)_2$ or $-COOC(CH_3)_3$ and $R^1$, $R^2$ and $R^3$ are all hydrogen, then $R^4$ is not propyl, butyl or hexyl, and

(g) when $R^6$ is -CONHphenyl not all of $R^1$, $R^2$, $R^3$ and $R^4$ are ethyl, or

B. when X is -CN then Y is an organic quaternary ammonium cation

other than tetramethylammonium, or is a cation comprising one or more hydrogen atoms co-ordinated to an organic base other than isopropylamine, t-butylamine, t-octylamine, diethylamine, triethylamine, pyridine, piperidine, pyrrolidine, benzamidine, aniline, N-methylaniline, o-toluidine or m-nitroaniline.

4. A compound according to Claim 3, wherein each of $R^1$, $R^2$, $R^3$, $R^4$ and $R^6$, when they contain carbon, contain less than 10 carbon atoms.

5. A compound according to Claim 3 or 4, wherein $R^4$ is branched alkyl C 3 to 6, phenyl or phenyl substituted by one or more or C 1 to 6 alkyl groups, $-NH_2$ or $-CF_3$ groups, or halogen atoms.

6. A compound according to any one of Claims 3 to 5, wherein $R^1$, $R^2$ and $R^3$ are each hydrogen or alkyl C 1 to 6.

7. A compound according to any one of Claims 3 to 6, wherein $R^6$ is a carboxylic acid group or a C 1 to 6 alkyl ester, or a C 1 to 6 alkyl substituted, or an unsubstituted, amide thereof.

8. A compound according to any one of Claims 3 to 7, wherein $R^4$ is phenyl optionally substituted by chlorine, bromine, iodine, methyl, $-CF_3$ or $-NH_2$, $R^3$ is hydrogen or methyl, $R^1$ and $R^2$ are chosen from hydrogen or methyl, and $R^6$ is $-CN$, $-CONH_2$, $-COOC_2H_5$, -COOi-propyl, -COOt-butyl or $-COOCH(CH_3)CH_2CH_3$.

9. A compound of formula Ic,

BAD ORIGINAL

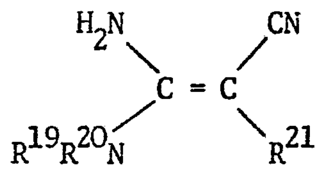

Ic

in which $R^{19}$ is hydrogen or methyl, $R^{20}$ is a group $R^{16}$ or a group

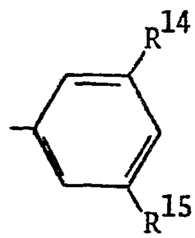 or 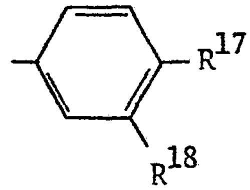

$R_{21}$ is $-CONH_2$ or -COOR,

R is straight or branched alkyl C 2 to 12,

$R^{14}$ and $R^{15}$, which may be the same or different, are each hydrogen, methyl, $-CF_3$ or nitro,

$R^{16}$ is branched chain alkyl C 3 to 6,

$R^{17}$ is chlorine or bromine, and

$R^{18}$ is hydrogen or methyl.

10. A compound of formula Id, Ie or If,

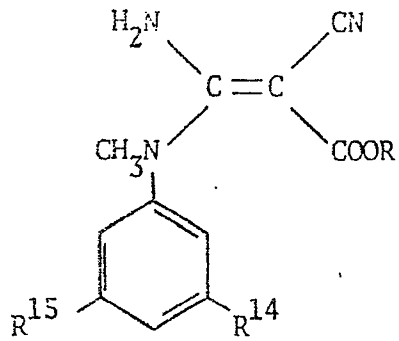

Id

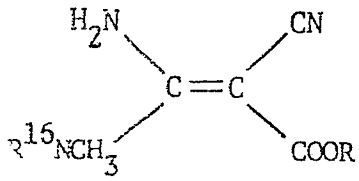

Ie

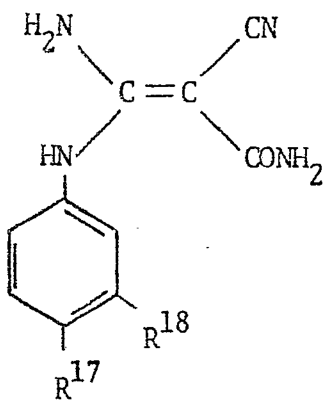

If

in which R, $R^{14}$, $R^{15}$, $R^{16}$, $R^{17}$ and $R^{18}$ are as defined in Claim 9.

11. 3-Amino-3-(4-bromophenylamino)-2-cyanopropenamide,

3-Amino-3-(4-chlorophenylamino)-2-cyanopropenamide,

3-Amino-3-(4-chloro-3-methylphenylamino)-2-cyanopropenamide,

Ethyl 3-amino-2-cyano-3-(N-methylphenylamino)propenoate,

Isopropyl 3-amino-2-cyano-3-(N-methylphenylamino)propenoate, or the dodecylguanidine salt of tricyanomethane.

12. A process for the production of a compound according to Claim 3, or a suitable derivative thereof, which comprises

(a) production of a compound of formula I in which X and Y together form a group of formula II by reacting a compound of formula III,

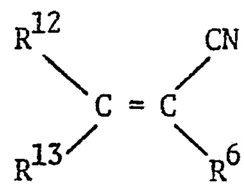

III

in which at least one of $R^{12}$ and $R^{13}$ is a leaving group, and the other of $R^{12}$ and $R^{13}$ may be $-NR^3R^4$ or $-NR^1R^2$ respectively,

with an amine of formula IV or V,

$R^3R^4NH$ IV

$R^1R^2NH$ V

in which $R^1$, $R^2$, $R^3$, $R^4$, $R^6$ and the provisos are as defined in claim 3, or

(b) production of a compound of formula I in which at least one of $R^1$, $R^2$, $R^3$ and $R^4$ represents alkyl, by alkylation of a corresponding compound of formula I in which at least one of $R^1$, $R^2$, $R^3$ and $R^4$ represents hydrogen, or

(c) production of a compound of formula I in which $R^3$ and $R^4$ or $R^1$ and $R^2$ are both hydrogen by subjecting to an elevated

temperature a compound of formula VI or VII,

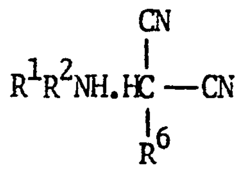

VI

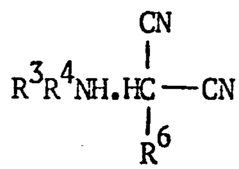

VII

in which $R^1$, $R^2$, $R^3$, $R^4$, $R^6$ and the provisos are as defined in claim 3,

(d) production of a compound of formula I in which X and Y together form a group of formula II and $R^6$ is $-CONH_2$, by selective hydrolysis of the corresponding compound of formula I in which $R^6$ is -CN,

(e) production of a compound of formula I in which X and Y together form a group of formula II and $R^6$ is $-C(=NH)-NHR^8$, by reacting a corresponding compound of formula I or III in which $R^6$ is -CN, with an amine of formula $R^8NH_2$ in which $R^8$ is as defined above, or

(f) production of a compound of formula I in which X is -CN and Y is a cation, by a metathetical process,

and where desired or necessary converting the resulting compound of formula I to a suitable derivative thereof or vice versa.

13. A method for combating fungus or weeds which comprises applying a composition according to Claim 1 or 2, or a compound according to

any one of Claims 3 to 11, to a locus infested, or liable to be infested, by fungal attack or by weeds.

European Patent Office

# EUROPEAN SEARCH REPORT

Application number 0010396

EP 79302156.9

| Category | DOCUMENTS CONSIDERED TO BE RELEVANT<br>Citation of document with indication, where appropriate, of relevant passages | Relevant to claim | CLASSIFICATION OF THE APPLICATION (Int. Cl. 3) |
|---|---|---|---|
| | Chemical Abstracts Vol.57 (1962), pp. 826, 827<br>-- | 3-12 | A 01 N 37/34//<br>C 07 C 121/28<br>C 07 C 121/407<br>C 07 C 121/417<br>C 07 C 121/45 |
| | DE - A1 - 2 800 764 (CIBA-GEIGY AG)<br>+ Claims 1 to 4 +<br>-- | 3-12 | |
| | DE - A1 - 2 601 052 (CIBA-GEIGY AG)<br>+ Claims 1 to 6 +<br>-- | 1-13 | TECHNICAL FIELDS SEARCHED (Int.Cl. 3) |
| | CH - A - 598 754 (CIBA-GEIGY AG)<br>+ Claims +<br>-- | 1,2 | A 01 N 37/00<br>C 07 C 121/00 |
| | CH - A - 532 357 (MONSANTO)<br>+ Claim II and subclaims +<br>---- | 1,2 | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 14-12-1979 | HLAVA |

EPO Form 1503.1 06.78